# EUROPEAN PATENT APPLICATION

(11) **EP 3 988 551 A1**
(43) Date of publication of application: **27.04.2022**
(21) Application number: 20827431.6
(22) Date of filing: 20.05.2020
(51) Int. Cl.: C07D 487/04, C07D 519/00, A61K 31/53, A61K 31/5377, A61P 35/00

(54) **CDK KINASE INHIBITOR**

(30) Priority: 18.06.2019 CN 201910529663
(71) Applicant: Beijing Benicall Biotech Co., Ltd., Beijing 100036 (CN)
(72) Inventor: PAN, Zhengying, Beijing 102206 (CN); ZHANG, Rui, Beijing 102206 (CN); QI, Zude, Beijing 102206 (CN)
(74) Representative: Murgitroyd & Company
(86) International application number: PCT/CN2020/091324
(87) International publication number: WO 2020/253458

(57) **Abstract**

Disclosed in the present application are a compound of general formula (I) capable of being used as a CDK kinase (in particular, CDK4/6 kinase) inhibitor, and a salt thereof, wherein all variates are defined as the present text. The compound can be used for treating or preventing diseases such as cancer. The present application further relates to a pharmaceutical composition comprising the compound of formula (I).

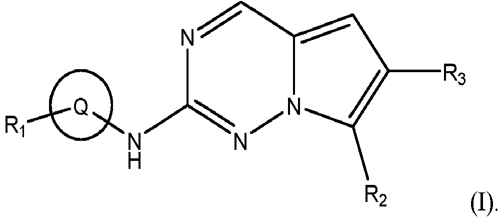

## Description

### TECHNICAL FIELD

The present invention relates to a compound that inhibits the activity of CDK kinase, and use of the above compound in the manufacture of a medicament for treating and/or preventing a cancer-related disease mediated by CDK kinase.

### BACKGROUND OF THE INVENTION

Cyclin-Dependent-Kinases (CDKs) are a group of serine/threonine protein kinases, which are closely related to important cellular processes such as cell cycle or transcription regulation. Studies have shown that, instead of exerting kinase activity *per se*, CDKs exert protein kinase activity by binding to cyclin to form a specific CDK-Cyclin complex, thereby possessing functions such as promoting phase transition of the cell cycle, initiating DNA synthesis, and regulating cellular transcription.

Currently, the CDK family comprises 13 members (CDK1 to CDK13). According to their intracellular functions, they are classified into two major categories: CDKs that control the cell cycle (CDK1, CDK2, CDK4, CDK6, etc.) and CDKs that control the transcription (CDK7, CDK9, etc.). There are 11 subtypes of cyclins, named after A-I, k, and T. Their expressions are transcriptionally regulated and fluctuate regularly during the cell cycle. In the CDK subtypes involved in cell cycle regulation, CDK4/6 plays an irreplaceable role. Cancer-related cell cycle mutations mainly exist in the G1 phase and the G1/S transition process. CDK4/6 binds to Cyclin D to form a complex with a kinase activity, which releases the bound transcription factor E2F through phosphorylation of the oncostatin Rb, initiating transcription of the genes related to the S phase, and prompting cells to pass through the checkpoint and transfer from the G1 phase to the S phase. The specific activation of CDK4/6 is closely related to the proliferation of certain tumors. There is an abnormal cyclin D-CDK4/6-INK4-Rb pathway in about 80% of human tumors. A change in this pathway may accelerate the progression of the G1 phase, resulting in accelerated proliferation and survival advantages of tumor cells. Therefore, it is a treatment strategy to interfere with the pathway, and CDK4/6 becomes one of the anti-tumor targets.

To date, more than 50 CDK inhibitors have been reported, some of which have potential anti-tumor activities. Some broad-spectrum CDK inhibitors have been developed as anti-tumor drugs, and some are undergoing preclinical or clinical trials. New CDK inhibitors are constantly being developed. Flavopiridol, also known as L86-8275 or HMR1275, a current inhibitor for CDK, is a representative of the first generation of CDK inhibitors. It has not entered Phase III clinical trial due to unobvious efficacy and high toxicity.

Recently, some pharmaceutical companies, including Pfizer, Eli Lilly, and Novartis, successively reported a series of CDK inhibitors with better selectivity, which are in clinical trials. Of particular concern are Palbociclib (PD-0332991) developed by Pfizer, Abemaciclib (LY2835219) developed by Eli Lilly, and Ribociclib (LEE011) developed by Novartis.

Palbociclib is a highly specific CDK4- (IC₅₀=0.011 µmol/L) and CDK6- (IC₅₀₌0.016 µmol/L) selective inhibitor developed by Pfizer (see Literatures 1 and 2). Palbociclib is inactive for 36 protein kinases including other CDK tyrosine/serine and threonine kinases. In February 2015, Palbociclib was approved by the US FDA for the treatment of patients with estrogen receptor-positive breast cancer. The successful listing of this compound once again set off a wave of development of CDK inhibitors.

Abemaciclib is an orally effective cyclin-dependent kinase (CDK) inhibitor, which targets the CDK4 (cyclin D1) and CDK6 (cyclin D3) cell cycle pathways, and has a potential anti-tumor activity. Abemaciclib specifically inhibits CDK4/6, thereby inhibiting phosphorylation of retinoblastoma (Rb) protein in the early G1 phase. Inhibition of Rb phosphorylation prevents CDK-mediated G1-S transition, such that the cell cycle arrests in the G1 phase, thereby inhibiting DNA synthesis and inhibiting the growth of cancer cells. In March 2017, Eli Lilly announced a successful Phase III clinical study of Abemaciclib in the treatment of breast cancer.

Ribociclib is another orally effective CDK4- and CDK6-selective inhibitor (with IC₅₀ of 10 and 39 nmol/L, respectively) (see Literatures 3 and 4). As expected, Ribociclib inhibits Rb phosphorylation, causes G0/G1 phase arrest, and induces senescence of tumor cells (including melanoma, breast cancer, liposarcoma, and neuroblastoma cells with B-Raf or N-Ras mutation). On March 14, 2017, Ribociclib was approved by the US FDA as a combination regimen with an aromatase inhibitor for the treatment of hormone receptor positive and human epidermal growth factor receptor 2 negative postmenopausal female patients.

At present, an increasing number of researches focus on this target in the world. CDK-targeting small molecule inhibitor (especially, CDK4/6 kinase inhibitor) drugs have a high development value. There is a large room for development, and it is of great significance for exploring new anti-tumor drugs in this field.

### Prior art literatures

Patent Literature 1: WO2003062236A1;
Patent Literature 2: WO2005005426A1;
Patent Literature 3: WO2011101409A1;
Patent Literature 4: CN103788100A.

### SUMMARY OF THE INVENTION

The problem to be solved by the invention

The objective of the present invention is to provide a kinase inhibitor compound against CDK.

### Means to solve the problem

The present invention provides a compound of Formula (I): 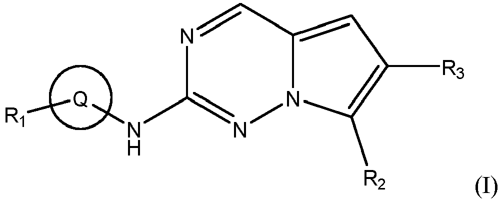 wherein,
Q is an optionally substituted 6- to 18-membered arylene group or an optionally substituted 5- to 18-membered heteroarylene group, wherein, when substituted, the substituent is selected from the group consisting of halo, hydroxy, C₁₋₆alkyl, C₁₋₆alkoxy, and halo-C₁₋₆alkyl;
R₁ is an optionally substituted 3- to 8-membered heterocyclyl group, an optionally substituted 6- to 14-membered fused heterocyclyl group, or an optionally substituted 6- to 12-membered spiro heterocyclyl group, wherein, when substituted, the substituent is selected from the group consisting of halo, hydroxy, C₁₋₆alkyl, C₁₋₆alkoxy, and halo-C₁₋₆alkyl;
R₂ is H, halo, an optionally substituted 3- to 10-membered cycloalkenyl group, an optionally substituted 3- to 10-membered heterocycloalkenyl group, an optionally substituted 3- to 10-membered cycloalkyl group, an optionally substituted 3- to 10-membered heterocycloalkyl group, an optionally substituted 6- to 18-membered aryl group, or an optionally substituted 5- to 18-membered heteroaryl group, wherein, when substituted, the substituent is selected from the group consisting of halo, hydroxy, C₁₋₆alkyl, C₁₋₆alkoxy, halo-C₁₋₆alkyl, C₁₋₆alkoxy-C₁₋₆alkoxy, and oxo;
R₃ is H, CN, -C(=O)-NR₄R₅, an optionally substituted 6- to 18-membered aryl group, an optionally substituted 5- to 18-membered heteroaryl group, or an optionally substituted 5- to 8-membered lactam group, wherein, when substituted, the substituent is selected from the group consisting of halo, hydroxy, C₁₋₆alkyl, C₁₋₆alkoxy, and halo-C₁₋₆alkyl;
R₄ and R₅ are each independently methyl or ethyl; and
when R₃ is -C(=O)-NR₄R₅, R₂ is an optionally substituted 3- to 10-membered cycloalkenyl group, an optionally substituted 3- to 10-membered heterocycloalkenyl group, an optionally substituted 3-to 10-membered heterocycloalkyl group connected to the non-R₂ structure in Formula (I) at a N atom, or an optionally substituted 6- to 18-membered aryl group, wherein, when substituted, the substituent is selected from the group consisting of halo, hydroxy, C₁₋₆alkyl, C₁₋₆alkoxy, halo-C₁₋₆alkyl, C₁₋₆alkoxy-C₁₋₆alkoxy, and oxo,
or a pharmaceutically acceptable salt thereof.

In a preferred embodiment,
Q is optionally substituted phenylene or optionally substituted pyridinylene, wherein, when substituted, the substituent is selected from the group consisting of halo, hydroxy, C₁₋₆alkyl, C₁₋₆alkoxy, and halo-C₁₋₆alkyl;
R₁ is an optionally substituted 3- to 8-membered heterocyclyl group, an optionally substituted 6- to 14-membered fused heterocyclyl group, or an optionally substituted 6- to 12-membered spiro heterocyclyl group, wherein, when substituted, the substituent is selected from C₁₋₆alkyl;
R₂ is selected from the group consisting of a halogen atom, optionally substituted cyclopentenyl, optionally substituted cyclohexenyl, optionally substituted cyclopentyl, optionally substituted cyclohexyl, optionally substituted oxacyclohexenyl, optionally substituted azacyclohexenyl, optionally substituted oxolanyl, optionally substituted azacyclopentyl, optionally substituted oxacyclohexyl, optionally substituted azacyclohexyl, optionally substituted phenyl, optionally substituted naphthyl, optionally substituted pyridinyl, optionally substituted thienyl, optionally substituted pyrazolyl, optionally substituted oxazolyl, optionally substituted isoxazolyl, and optionally substituted quinolyl, wherein, when substituted, the substituent is selected from the group consisting of halo, hydroxy, C₁₋₆alkyl, C₁₋₆alkoxy, halo-C₁₋₆alkyl, and oxo;
R₃ is H, -CN, -C(=O)-NR₄R₅, optionally substituted phenyl, naphthyl, pyrazolyl, pyridinyl, thienyl, oxazolyl, isoxazolyl, pyrimidinyl, imidazolyl, pyrrolyl, 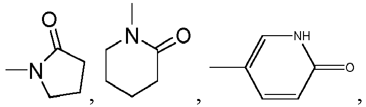 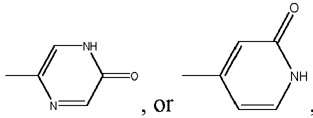 , wherein, when substituted, the substituent is selected from the group consisting of halo, hydroxy, C₁₋₆alkyl, C₁₋₆alkoxy, and halo-C₁₋₆alkyl; and
both R₄ and R₅ are methyl.

In another preferred embodiment, Q is phenylene or

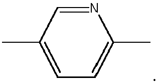

In another preferred embodiment, R₁ is an optionally substituted group as below, wherein, when substituted, the substituent is selected from C₁₋₆alkyl:

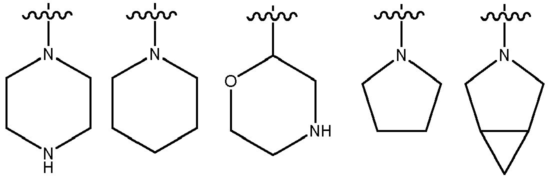

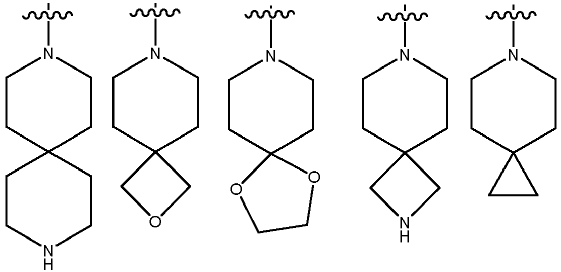

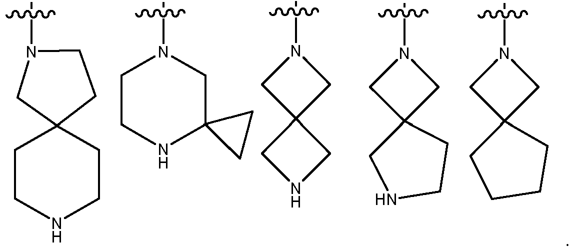

In another preferred embodiment, the C₁₋₆alkyl is methyl or ethyl.

In another preferred embodiment, R₂ is halo or an optionally substituted group as below, wherein, when substituted, the substituent is selected from C₁₋₆alkyl:

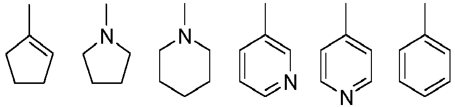

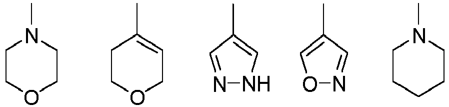

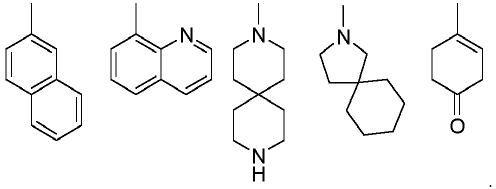

In another aspect of the present invention, each Example compound in Table 1 or a pharmaceutically acceptable salt thereof is provided.

In another aspect of the present invention, the compound of the present invention is used for inhibiting the activity of CDK kinase; in particular, the compound of the present invention is used for inhibiting the activity of CDK4/6 kinase.

In another aspect of the present invention, there is provided a pharmaceutical composition comprising an effective amount of the above compound of the present invention, and a pharmaceutically acceptable carrier or excipient.

In another aspect of the present invention, there is provided use of a compound of the present invention or a pharmaceutically acceptable salt thereof in the manufacture of a medicament for treating and/or preventing a cancer-related disease mediated by CDK kinase (particularly, CDK4/6 kinase), wherein the cancer-related disease is selected from brain tumor, lung cancer, squamous cell carcinoma, bladder cancer, stomach cancer, ovarian cancer, peritoneal cancer, pancreatic cancer, breast cancer, head and neck cancer, cervical cancer, endometrial cancer, rectal cancer, liver cancer, kidney cancer, esophageal adenocarcinoma, esophageal squamous cell carcinoma, prostate cancer, female reproductive tract cancer, carcinoma *in situ,* lymphoma, neurofibroma, thyroid cancer, bone cancer, skin cancer, brain cancer, colon cancer, testicular cancer, gastrointestinal stromal tumor, prostate tumor, mast cell tumor, multiple myeloma, melanoma, glioma, or sarcoma.

### The effect of the invention

The compound of the present invention can inhibit the activity of CDK kinase (particularly, CDK4/6 kinase), and can be used in the treatment of cancer.

### DETAILED DESCRIPTION OF THE INVENTION

Unless defined otherwise, all technical and scientific terms used herein have the same meaning as is commonly understood by one of skill in the art to which the claimed subject matter belongs.

"C₁₋₆alkyl" refers to an alkyl group with 1-6 carbon atoms, including methyl, ethyl, propyl, butyl, pentyl, and hexyl, including all possible isomeric forms, such as n-propyl, isopropyl, n-butyl, isobutyl, sec-butyl, tert-butyl, etc. "C₁₋₆alkyl" includes all sub-ranges contained therein, such as C₁₋₂alkyl, C₁₋₃alkyl, C₁₋₄alkyl, C₁₋₅alkyl, C₂₋₅alkyl, C₃₋₅alkyl, C₄₋₅alkyl, C₃₋₄alkyl, C₃₋₅alkyl, and C₄₋₅alkyl.

"Aryl" and "heteroaryl" include monocyclic or fused polycyclic (i.e., rings which share adjacent pairs of ring atoms) groups. Examples of "aryl" include, but are not limited to, phenyl, naphthalenyl, phenanthrenyl, anthracenyl, fluorenyl, and indenyl. Examples of "heteroaryl" include: 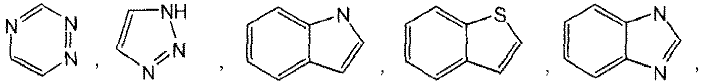 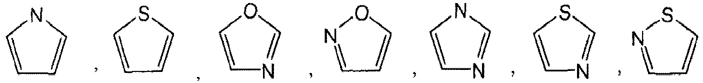 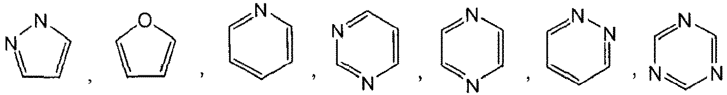 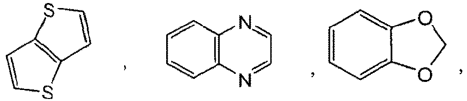 and the like.

The term "6- to 18-membered arylene", as described herein, refers to a divalent group derived from an aromatic hydrocarbon with 6-18 carbon atoms by abstraction of two hydrogen atoms, including, for example, "6- to 14-membered arylene", "6- to 10-membered arylene", etc. Examples include, but are not limited to, phenylene, naphthylene, anthrylene, and the like.

The term "5- to 18-membered heteroarylene", as described herein, refers to a divalent group derived from a 5- to 18-membered heteroaromatic hydrocarbon by abstraction of two hydrogen atoms, including, for example, "5- to 14-membered heteroarylene", "5- to 10-membered heteroarylene", etc. Examples include, but are not limited to, furylene, thienylene, pyrrolylene, imidazolylene, thiazolylene, pyrazolylene, oxazolylene, isoxazolylene, isothiazolylene, pyridinylene, pyrazinylene, pyridazinylene, pyrimidinylene, and the like, preferably pyridinylene, pyrazolylene or thienylene, particularly preferably pyridinylene, and most preferably pyridinylene in which the nitrogen atom is located at the 3-position of the bonding position of R1.

The term "3- to 8-membered heterocyclyl", as described herein, includes, for example, "3- to 7-membered heterocyclyl", "3- to 6-membered heterocyclyl", "4- to 7-membered heterocyclyl", "4- to 6-membered heterocyclyl", "5- to 7-membered heterocyclyl", "5- to 6-membered heterocyclyl", "6-membered heterocyclyl", etc. Specific examples include, but are not limited to, aziridinyl, 2H-aziridinyl, diaziridinyl, 3H-diazirenyl, azetidinyl, 1,4-dioxanyl, 1,3-dioxanyl, 1,3-dioxolanyl, 1,4-dioxacyclohexadienyl, tetrahydrofuryl, dihydropyrrolyl, pyrrolidinyl, imidazolidinyl, 4,5-dihydroimidazolyl, pyrazolidinyl, 4,5-dihydropyrazolyl, 2,5-dihydrothienyl, tetrahydrothienyl, 4,5-dihydrothiazolyl, piperidinyl, piperazinyl, morpholinyl, 4,5-dihydrooxazolyl, 4,5-dihydroisoxazolyl, 2,3-dihydroisoxazolyl, 2H-1,2-oxazinyl, 6H-1,3-oxazinyl, 4H-1,3-thiazinyl, 6H-1,3-thiazinyl, 2H-pyranyl, 2H-pyran-2-onyl, 3,4-dihydro-2H-pyranyl, and the like, preferably "5- to 6-membered heterocyclyl".

The term "6- to 14-membered fused heterocyclyl", as described herein, includes, for example, "6- to 11-membered fused heterocyclyl", "6- to 10-membered fused heterocyclyl", "7- to 10-membered fused heterocyclyl", "9- to 10-membered fused heterocyclyl", etc. Specific examples include, but are not limited to:

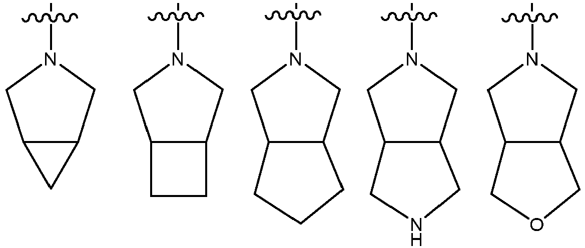

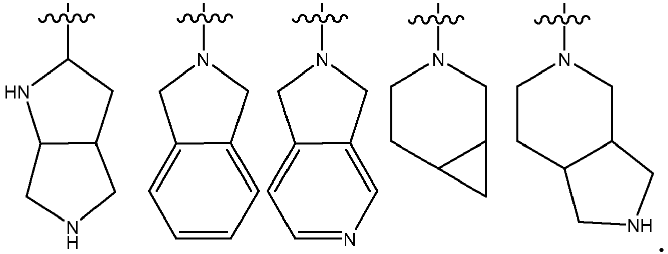

The term "6- to 12-membered spiro heterocyclyl", as described herein, refers to a cyclic structure with 6-12 ring atoms containing at least one heteroatom, which is formed from at least two rings sharing one atom. The heteroatom is selected from N, S, O, CO, SO and/or SO₂, etc. The term includes, for example, "6- to 11-membered spiro heterocyclyl", "7- to 11-membered spiro heterocyclyl", "7- to 10-membered spiro heterocyclyl", "7- to 9-membered spiro heterocyclyl", "7-to 8-membered spiro heterocyclyl", etc. Examples include, but are not limited to, for example:

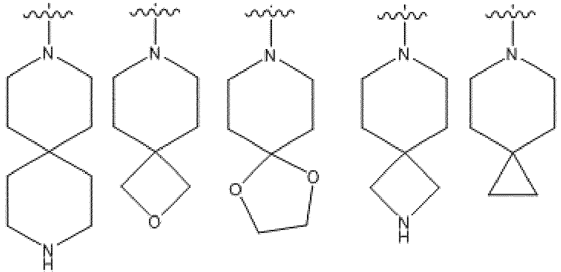

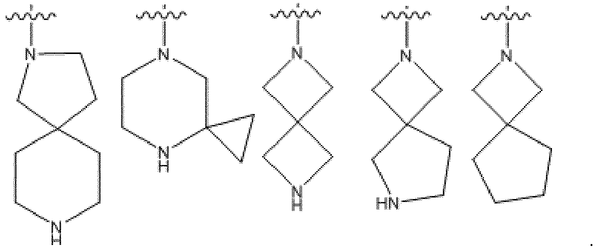

The term "3- to 10-membered cycloalkenyl", as described herein, refers to a cyclic alkenyl group derived from a cyclic monoolefine moiety with 3 to 10 carbon atoms by abstraction of one hydrogen atom, including, for example, "3- to 8-membered cycloalkenyl", "4- to 6-membered cycloalkenyl", etc. Examples include, but are not limited to, cyclopropenyl, cyclobutenyl, cyclopentenyl, cyclohexenyl, cycloheptenyl, cyclooctenyl, cyclononenyl, cyclodecenyl, and the like.

The term "3- to 10-membered heterocycloalkenyl", as described herein, refers to a heterocycloalkenyl group derived from a 3- to 10-membered cyclic monoolefine moiety containing a heteroatom by abstraction of one hydrogen atom, including, for example, "3- to 8-membered heterocycloalkenyl", "4- to 6-membered heterocycloalkenyl", etc. Examples include, but are not limited to:

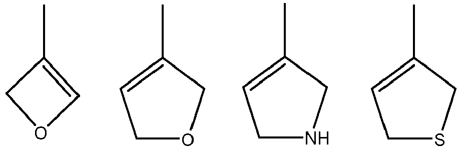

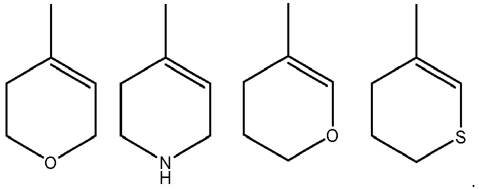

The term "3- to 10-membered cycloalkyl", as described herein, refers to a cyclic alkyl group derived from a cycloalkane moiety with 3 to 10 carbon atoms by abstraction of one hydrogen atom, including, for example, "3- to 8-membered cycloalkyl", "4- to 6-membered cycloalkyl", etc. Examples include, but are not limited to, cyclopropanyl, cyclobutanyl, cyclopentanyl, cyclohexanyl, cycloheptanyl, cyclooctanyl, cyclononanyl, cyclodecanyl, and the like.

The term "3- to 10-membered heterocycloalkyl", as described herein, refers to a heterocycloalkyl group derived from a 3- to 10-membered heterocycloalkane moiety containing a heteroatom by abstraction of one hydrogen atom, including, for example, "3- to 8-membered heterocycloalkyl", "4-to 6-membered heterocycloalkyl", etc. Examples include, but are not limited to, aziridinyl, azetidinyl, azacyclopentanyl, azacyclohexanyl, azepanyl, azocanyl, azoninyl, and the like.

The term "oxo", as described herein, refers to a substituent in which an oxygen atom is bonded to a carbon atom or a nitrogen atom. Specific examples of the structure in which an oxygen atom is bonded to a carbon atom include carbonyl, and specific examples of the group in which an oxygen atom is bonded to a nitrogen atom include N-oxide.

"Halo" refers to fluoro, chloro, bromo and iodo. "C₁₋₆alkoxy" refers to (C₁₋₆alkyl)O-, where the C₁₋₆alkyl is as defined herein. "Halo-C₁₋₆alkyl" refers to halo-(C₁₋₆alkyl)-, where the C₁₋₆alkyl is as defined herein. Halo-C₁₋₆alkyl includes perhalogenated C₁₋₆alkyl, in which all hydrogen atoms in the C₁₋₆alkyl are replaced with halogen, such as -CF₃, -CH₂CF₃, -CF₂CF₃, -CH₂CH₂CF₃, or the like.

The term "5- to 8-membered lactam group", as described herein, refers to a group derived from a lactam containing a -C(=O)-N group in the ring by abstraction of one hydrogen atom, including, for example, "5- to 7-membered lactam group". Examples include, but are not limited to:

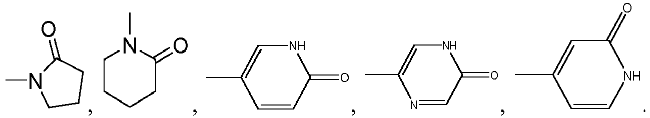

The compound of the present invention can be prepared into or used as a pharmaceutically acceptable salt thereof. This can be done by any salt-forming means known in the art. For example, the pharmaceutically acceptable salt can be an acid addition salt, such as an inorganic acid addition salt or an organic acid addition salt. For example, the pharmaceutically acceptable salt can also be a salt formed by replacing an acidic proton in the compound with a metal ion, or a salt formed by coordination of the compound with an organic base or an inorganic base.

The term "pharmaceutically acceptable", as used herein with respect to a formulation, composition or ingredient, means that it has no persistent detrimental effect on the general health of the subject being treated or does not abrogate the biological activity or properties of the compound, and is relatively nontoxic.

The terms "effective amount" or "therapeutically effective amount", as used herein, refer to a sufficient amount of an agent or a compound being administered, which will relieve to some extent one or more of the symptoms of the disease or condition being treated. The outcome can be reduction and/or alleviation of the signs, symptoms, or causes of a disease, or any other desired alteration of a biological system. For example, an "effective amount" for therapeutic uses is an amount required to provide a clinically significant decrease in disease symptoms without undue adverse side effects. An appropriate "effective" amount in any individual case can be determined using techniques, such as a dose escalation study. The term "therapeutically effective amount" includes, for example, a prophylactically effective amount. An "effective amount" of a compound disclosed herein is an amount effective to achieve a desired pharmacologic effect or therapeutic improvement without undue adverse side effects. It is understood that "an effect amount" or "a therapeutically effective amount" can vary from subject to subject, due to variations in metabolism of the compound, age, weight, general condition of the subject, the condition being treated, the severity of the condition being treated, and the judgment of the prescribing physician. By way of example only, therapeutically effective amounts may be determined by routine experimentation, including, but not limited to, a dose escalation clinical trial.

The terms "inhibit", "inhibiting", and "inhibitor" for a kinase, as used herein, mean inhibition of the activity of CDK4/6 kinase.

Regarding general methods for preparing the compound as disclosed herein, reference can be made to reactions known in the art. Those skilled in the art can reasonably select appropriate reagents and conditions to adjust or modify the reactions known in the art, for introducing the individual moieties in the structure of the compound as disclosed herein.

The starting material used for the synthesis of the compounds described herein may be synthesized or can be obtained from commercial sources, such as, but not limited to, Aldrich Chemical Co. (Milwaukee, Wisconsin), Bachem (Torrance, California), or Sigma Chemical Co. (St. Louis, Mo.). The compounds described herein, and other related compounds having different substituents, can be synthesized using techniques and materials known to those of skill in the art, such as described, for example, in March, ADVANCED ORGANIC CHEMISTRY, 4th Ed., (Wiley 1992); Carey and Sundberg, ADVANCED ORGANIC CHEMISTRY, 4th Ed., Vols. A and B (Plenum 2000, 2001); Green and Wuts, PROTECTIVE GROUPS IN ORGANIC SYNTHESIS, 3rd Ed., (Wiley 1999); Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991); and Larock's Comprehensive Organic Transformations (VCH Publishers Inc., 1989) (incorporated herein by reference in their entirety).

The products of the reactions may be isolated and purified, if desired, using conventional techniques, including, but not limited to, filtration, distillation, crystallization, chromatography, and the like. Such products may be characterized using conventional means, including physical constants and spectral data.

The compound described herein can be prepared into a single isomer or a mixture of isomers by the synthetic methods described herein.

The compounds described herein can possess one or more stereocenters and each center exists in the R or S configuration. The compounds presented herein include all diastereomeric, enantiomeric, and epimeric forms as well as appropriate mixtures thereof. Stereoisomers may be obtained, if desired, by methods known in the art, for example, separation of stereoisomers by a chiral chromatographic column.

Diasteromeric mixtures can be separated into their individual diastereomers on the basis of their differences in physicochemical properties by known methods, for example, by chromatography and/or fractional crystallization. In one embodiment, enantiomers can be separated by a chiral chromatographic column. In some other embodiments, enantiomers can be separated by converting the enantiomeric mixture into a diastereomeric mixture by reaction with an appropriate optically active compound (e.g., alcohol), separating the diastereomers, and converting (e.g., hydrolyzing) the individual diastereomers to the corresponding pure enantiomers. All such isomers, including diastereomers, enantiomers, and mixtures thereof, are considered as components of the compositions described herein.

The methods and formulations described herein include the use of N-oxides, crystalline forms (also known as polymorphs), or pharmaceutically acceptable salts of compounds described herein, as well as active metabolites of these compounds having the same type of activity. In some situations, compounds may exist as tautomers. All tautomers are included within the scope of the compounds presented herein. In addition, the compounds described herein can exist in unsolvated as well as solvated forms with pharmaceutically acceptable solvents such as water, ethanol, or the like. The solvated forms of the compounds presented herein are also considered to be disclosed herein.

An unoxidized form can be prepared from N-oxides by treating with a reducing agent, such as, but not limited to, sulfur, sulfur dioxide, triphenyl phosphine, lithium borohydride, sodium borohydride, phosphorus trichloride, tribromide, or the like, in a suitable inert organic solvent, such as, but not limited to, acetonitrile, ethanol, aqueous dioxane, or the like, at 0 to 80 °C.

In some embodiments, compounds described herein are prepared into prodrugs. A "prodrug" refers to an agent that is converted into the parent drug *in vivo.* Prodrugs are often useful because, in some situations, they are easier to administer than the parent drug. For example, in some cases, an active compound itself is difficult to be bioavailable by oral administration, and a prodrug can be used to achieve this goal. The prodrug may have improved solubility in pharmaceutical compositions over the parent drug. Prodrugs may be designed as reversible drug derivatives, to enhance drug transport to site-specific tissues. In some embodiments, the design of a prodrug increases the effective water solubility. See, e.g., Fedorak, et al., Am. J. Physiol, 269: G210-218(1995); McLoed, et al., Gastroenterol, 106: 405-413(1994); Hochhaus, et al., Biomed. Chrom., 6:283-286 (1992); J. Larsen and H. Bundgaard, Int. J. Pharmaceutics, 37, 87(1987); J. Larsen, et al., Int. J. Pharmaceutics, 47, 103 (1988); Sinkula, et al., J. Pharm. Sd., 64:181-210 (1975); T. Higuchi and V. Stella, Pro-drugs as Novel Delivery Systems, the A.C.S. Symposium Series, Vol. 14; and Edward B. Roche, Carriers in Drug Design, American Pharmaceutical Association and Pergamon Press, 1987, all incorporated herein by reference in their entirety. An non-limiting example of a prodrug would be a compound as described herein which is administered as an ester (the "prodrug") to facilitate transmittal across a cell membrane where water solubility is detrimental to mobility but which then is metabolically hydrolyzed to the carboxylic acid, the active entity, once inside the cell where water solubility is beneficial. A further example of a prodrug might be a short peptide (polyamino acid) bonded to an acid group where the peptide is metabolized to reveal the active moiety. In certain embodiments, upon *in vivo* administration, a prodrug is chemically converted to the biologically, pharmaceutically or therapeutically active form of the compound. In certain embodiments, a prodrug is enzymatically metabolized by one or more steps or processes to the biologically, pharmaceutically or therapeutically active form of the compound. A pharmaceutically active compound can be modified to produce a prodrug such that the active compound will be regenerated upon *in vivo* administration. The prodrug can be designed to alter the metabolic stability or the transport characteristics of a drug, to mask side effects or toxicity, to improve the effect of a drug or to alter other characteristics or properties of a drug. Based on knowledge of pharmacodynamic processes and drug metabolism *in vivo,* once a pharmaceutically active compound is known, those skilled in the art can design prodrugs of the compound (see, for example, Nogrady (1985) Medicinal Chemistry A Biochemical Approach, Oxford University Press, New York, pages 388-392; Silverman (1992), The Organic Chemistry of Drug Design and Drug Action, Academic Press, Inc., San Diego, pages 352-401; and Saulnier, et al., (1994), Bioorganic and Medicinal Chemistry Letters, Vol. 4, p. 1985).

Prodrug forms of the compounds described herein, wherein the prodrug is metabolized *in vivo* to produce a derivative as set forth herein, are included within the scope of the claims. In some cases, some of the compounds described herein may be prodrugs or other derivatives of active compounds.

The compounds described herein encompass compounds comprising isotopes, which are identical to those recited herein in molecular formula and structural formula, but for the fact that one or more atoms are replaced by a nuclide that belongs to the same element but has an atomic mass or mass number different from the atomic mass or mass number usually found in nature. For example, when hydrogen is present at any position in the compound described herein, it also includes the case where an isotope of hydrogen (e.g., protium, deuterium, tritium, or the like) occurs at that position. Examples of isotopes that can be incorporated into the compounds described herein include, but are not limited to, isotopes of hydrogen, carbon, nitrogen, oxygen, fluorine and chlorine, such as, for example, ²H, ³H, ¹³C, ¹⁴C, ¹⁵N, ¹⁸O, ¹⁷O, ³⁵S, ¹⁸F, ³⁶Cl, respectively. The compounds described herein that comprise certain isotopes (e.g., radioactive isotopes such as ³H and ¹⁴C) are useful in drug and/or substrate tissue distribution assays.

In additional or further embodiments, the compounds described herein are metabolized upon administration to an organism in need thereof to produce a metabolite that is then used to produce a desired effect, including a desired therapeutic effect.

Compounds described herein may be formed as, and/or used as, pharmaceutically acceptable salts. The types of pharmaceutical acceptable salts, include, but are not limited to: (1) acid addition salts, formed by reacting the free base form of the compound with a pharmaceutically acceptable inorganic acid such as hydrochloric acid, hydrobromic acid, sulfuric acid, nitric acid, phosphoric acid, metaphosphoric acid, or the like; or with an organic acid such as acetic acid, propionic acid, hexanoic acid, cyclopentanepropionic acid, glycolic acid, pyruvic acid, lactic acid, malonic acid, adipic acid, sebacic acid, succinic acid, malic acid, maleic acid, fumaric acid, trifluoroacetic acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, toluenesulfonic acid, 2-naphthalenesulfonic acid, 4-methylbicyclo-[2.2.2]oct-2-ene-1-carboxylic acid, glucoheptonic acid, hippuric acid, gentisic acid, 4,4'-methylenebis-(3-hydroxy-2-ene-1-carboxylic acid), nicotinic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, or the like; (2) salts formed when an acidic proton present in the parent compound either is replaced by a metal ion, e.g., an alkali metal ion (e.g. lithium, sodium, or potassium), an alkaline earth ion (e.g. magnesium or calcium), or an aluminum ion; or (3) salts formed by coordination with an organic or inorganic base. Acceptable organic bases include ethanolamine, diethanolamine, triethanolamine, trimethylamine, N-methylglucamine, etc. Acceptable inorganic bases include aluminum hydroxide, calcium hydroxide, potassium hydroxide, sodium carbonate, sodium hydroxide, etc.

The corresponding counterions of the pharmaceutically acceptable salts may be analyzed and identified using various methods, including, but not limited to, ion exchange chromatography, ion chromatography, capillary electrophoresis, inductively coupled plasma, atomic absorption spectroscopy, mass spectrometry, or any combination thereof.

The salts can be recovered by using at least one of the following techniques: filtration, precipitation with a non-solvent followed by filtration, evaporation of the solvent, or, in the case of aqueous solutions, lyophilization.

It should be understood that a reference to a pharmaceutically acceptable salt includes the solvent addition forms or crystal forms thereof, particularly solvates or polymorphs. Solvates contain either stoichiometric or non-stoichiometric amounts of a solvent, and may be formed during the process of crystallization with pharmaceutically acceptable solvents such as water, ethanol, and the like. Hydrates are formed when the solvent is water, or alcoholates are formed when the solvent is alcohol. Solvates of the compounds described herein can be conveniently prepared or formed during the processes described herein. In addition, the compounds provided herein can exist in unsolvated as well as solvated forms. In general, the solvated forms are considered equivalent to the unsolvated forms for the purposes of the compounds and methods provided herein.

Compounds described herein may be in various forms, including, but not limited to, amorphous forms, sphere-shaped forms and nano-particulate forms. In addition, compounds described herein include crystalline forms, also known as polymorphs. Polymorphs include the different crystal packing arrangements of the same elemental composition of a compound. Polymorphs usually have different X-ray diffraction patterns, infrared spectra, melting points, density, hardness, crystal shapes, optical and electrical properties, stability, and solubility. Various factors such as the recrystallization solvent, rate of crystallization, and storage temperature may cause a single crystal form to dominate.

The screening and characterization of the pharmaceutically acceptable salts, polymorphs, and/or solvates may be accomplished using a variety of techniques, including, but not limited to, thermal analysis, x-ray diffraction, spectroscopy, vapor sorption, and microscopy. Thermal analysis methods address thermo chemical degradation or thermo physical processes, including, but not limited to, polymorphic transitions, and such methods can be used to analyze the relationships between polymorphic forms, and determine weight loss, to find the glass transition temperature, or for excipient compatibility studies. Such methods include, but are not limited to, Differential Scanning Calorimetry (DSC), Modulated Differential Scanning Calorimetry (MDCS), Thermogravimetric Analysis (TGA), and Thermogravimetric and Infrared Analysis (TG/IR). X-ray diffraction methods include, but are not limited to, single crystal and powder diffractometers and synchrotron sources. The various spectroscopic techniques used include, but are not limited to, Raman, FTIR, UVIS, and NMR (liquid and solid state). The various microscopy techniques include, but are not limited to, Polarized Light Microscopy, Scanning Electron Microscopy (SEM) with Energy Dispersive X-Ray Analysis (EDX), Environmental Scanning Electron Microscopy with EDX (in gas or water vapor atmosphere), IR Microscopy, and Raman Microscopy.

The carrier herein includes conventional diluents, excipients, fillers, binders, wetting agents, disintegrants, absorption enhancers, surfactants, adsorption carriers, lubricants, and, if necessary, flavoring agents, sweeteners, or the like, which are commonly used in the field of pharmacy. The medicament of the present invention can be prepared into various forms such as tablets, powders, granules, capsules, oral liquids, and injections. The above medicaments in various dosage forms can be prepared by conventional methods in the field of pharmacy.

As used herein, the IC₅₀ refers to an amount, concentration or dosage of a particular test compound that achieves a 50% inhibition of a maximal response, such as inhibition of CDK kinase, such as CDK4/6 kinase, in an assay that measures such response.

### EXAMPLES

The following specific and non-limiting Examples are to be construed as merely illustrative, and do not limit the present disclosure in any way. Without further elaboration, it is believed that those skilled in the art can, based on the description herein, utilize the present invention to its fullest extent.

### Synthesis of Compounds

The following abbreviations are used in the following synthesis schemes:
Boc: t-butoxycarbonyl;
Et: ethyl;
H: hour;
rt/RT: room temperature;
Me: methyl;
MeOH: methanol;
DIPEA: N,N-diisopropylethylamine;
DCM: dichloromethane;
NMP: N-methylpyrrolidone;
TEA: triethylamine;
TFA: trifluoroacetic acid;
THF: tetrahydrofuran;
HATU: 2-(7-benzotriazole oxide)-N,N,N',N'-tetramethyluronium hexafluorophosphate;
DMF: dimethylformamide;
NBS: N-bromosuccinimide;
NIS: N-iodosuccinimide;
DPPF PdCl₂: [1,1'-bis(diphenylphosphino)ferrocene]palladium dichloride;
XantPhos: 4,5-bisdiphenylphosphino-9,9-dimethylxanthene;
Pd₂(dba)₃: tris(dibenzylideneacetone)dipalladium.

### Synthesis Scheme I

### Step 1:

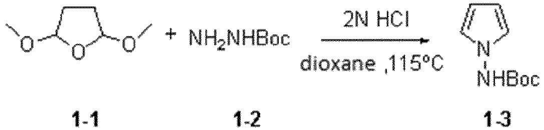

To a 1 L two-necked flask were added 100 g of Compound 1-1 and 91 g of Compound 1-2, and then 500 mL of dioxane was added as a solvent. A water separator and a condenser were connected. 9 mL of a hydrochloric acid solution (2 mol/L) was added. The mixture was heated to 115 °C and reacted for 1 h. Then, 2 mL of an HCl solution (2 mol/L) was added, and a reaction was conducted overnight at a controlled temperature of 95 °C. After the reaction was completed, the reaction mixture was cooled to room temperature. A saturated sodium bicarbonate solution was added to adjust the pH to an alkaline value. The mixture was concentrated to remove dioxane, and extracted with ethyl acetate. The resultant organic layer was dried over anhydrous sodium sulfate, filtered, concentrated, and purified by column chromatography (gradient elution, acetone : n-hexane = 1:8 to 1:5) to give 40 g of the product.

### Step 2:

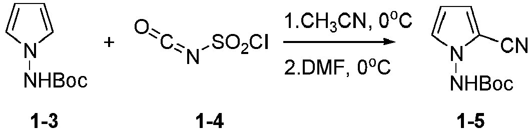

To a two-necked flask was added 40 g of Compound 1-3, and then 250 mL of acetonitrile was added for (ultrasonic) dissolution. Under the protection of argon, 20 mL of Compound 1-4 was slowly added dropwise in an ice-salt bath, and a reaction was conducted for 1 h. After the reaction was completed, 40 mL of DMF was added and a reaction was conducted for 1 h while stirring in the ice-salt bath. After the reaction was completed, the reaction mixture was poured into ice water, and extracted with ethyl acetate. The resultant organic layer was dried over anhydrous sodium sulfate, filtered, concentrated, and purified by column chromatography (gradient elution, ethyl acetate : n-hexane = 1:10 to 1:5) to give 3 8 g of the product.

### Step 3:

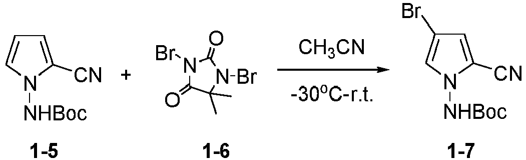

To a 1 L flask was added 38 g of Compound **1-5,** and then 665 mL of acetonitrile was added as a solvent. The temperature was decreased to -30 °C under the protection of argon. Then, 27.5 g of Compound **1-6** was slowly added, and reacted for 5 min while stirring. The reaction was warmed to room temperature for 2 h. After the reaction was completed, ethyl acetate and saturated brine were added for extraction. The resultant organic layer was dried over anhydrous sodium sulfate, filtered, concentrated, and purified by column chromatography (gradient elution, ethyl acetate : n-hexane = 1:10 to 1:5) to give 47 g of the product.

### Step 4:

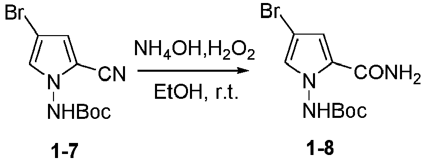

To a 2 L flask was added 47 g of Compound **1-7,** and then 940 mL of ethanol was added as a solvent. 470 mL of aqueous ammonia was added, and 83.5 mL of aqueous hydrogen peroxide (30%) was slowly added dropwise at room temperature. After addition, the mixture was stirred overnight. After the reaction was completed, the mixture was concentrated to remove ethanol and filtered to give a light yellow solid. The solid was washed with pure water, followed by methyl tert-butyl ether and n-hexane (1:1) to a white solid, and dried *in vacuo* to give 37 g of the product.

### Step 5:

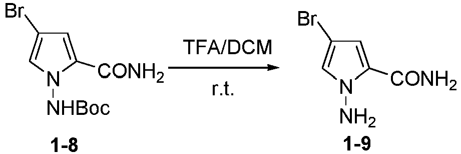

To a 100 mL flask was added 6 g of Compound **1-8,** and then 10 mL of dichloromethane was added for dissolution. Then, 20 mL of trifluoroacetic acid was added dropwise, and reacted for 2 h while stirring at room temperature. After the reaction was completed, the mixture was concentrated to remove the solvent and excess trifluoroacetic acid, adjusted to pH 7-8 with saturated sodium bicarbonate, and extracted with ethyl acetate. The resultant organic layer was dried over anhydrous sodium sulfate, filtered, and concentrated to give 3.6 g of the product.

### Step 6:

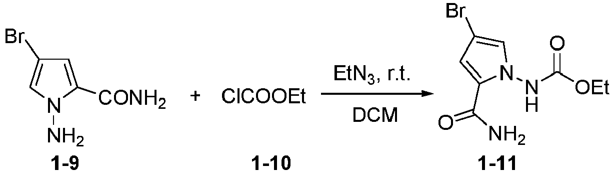

To a 250 mL flask were added 3 g of Compound **1-9,** 100 mL of dichloromethane, and then 2.4 g of Compound **1-10.** After stirring at room temperature, triethylamine (4.5 g) was slowly added dropwise. Upon completion of the reaction as monitored by TLC, the reaction mixture was concentrated to be ready for direct use.

### Step 7:

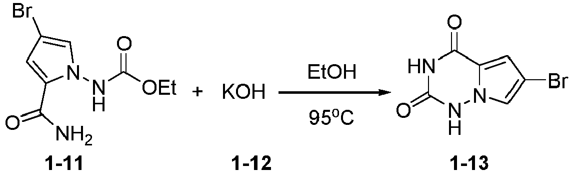

To a 250 mL flask was added the crude Compound **1-11** obtained from Step 6. Then, 100 mL of ethanol was added as a solvent, and 1.7 g of potassium hydroxide was added. The mixture was heated to reflux overnight. After the reaction was completed, acetic acid was added to adjust the pH to 5-6. Solids were precipitated out, and filtered. The filter cake was washed with water, followed by a small amount of diethyl ether, and dried under infrared light to give 2.5 g of the product.

### Step 8:

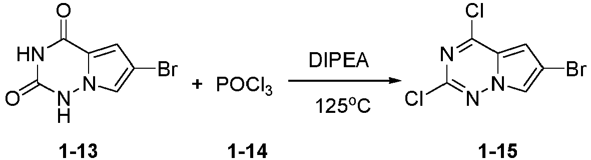

To a 100 mL pressure-resistant tube was added 2.5 g of Compound **1-13,** and then 20 mL of Compound **1-14** and 10 mL of DIPEA were added. The mixture was heated to 125 °C and reacted for 48 h. After the reaction was completed, the reaction mixture was concentrated to remove excess phosphorus oxychloride and DIPEA. 100 mL of ethyl acetate was added for dissolution and dilution. A small number of ice cubes were added in an ice-water bath, and then a saturated sodium bicarbonate solution was slowly added dropwise to adjust the pH to 7-8. A saturated sodium chloride solution was added, and the mixture was extracted with ethyl acetate. The resultant organic layer was dried, filtered, concentrated, and purified by column chromatography to give 1.5 g of the product.

### Step 9:

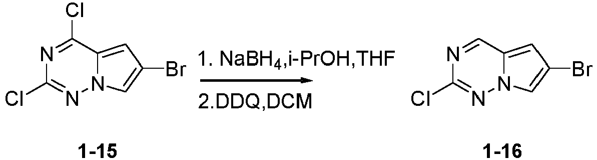

To a 100 mL flask were added 3.9 g of Compound **1-15** and 0.9 g of sodium borohydride, and then 40 mL of THF was added as a solvent. After 2 mL of isopropanol was added, a reaction was conducted for 1 h while stirring at room temperature. After the reaction was completed, the reaction mixture was filtered to remove solids, and washed with DCM. The filtrate was concentrated to remove excess THF, isopropanol, and DCM. Then, DCM as a solvent and 5 g of DDQ were added, and reacted at room temperature for 30 min. After the reaction was completed, the reaction mixture was filtered to remove solids. A saturated sodium bicarbonate solution was added to the filtrate, which was extracted with DCM, dried, filtered, concentrated, and purified by column chromatography to give 3 g of the product.

### Step 10:

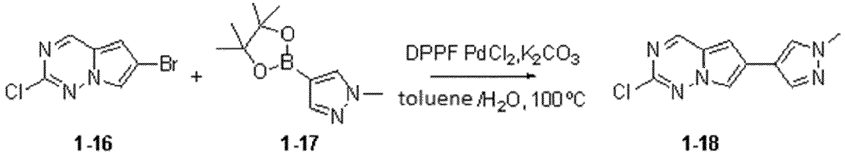

To a 50 mL flask were added 1 g of Compound **1-16,** 0.9 g of Compound **1-17,** 0.3 g of DPPF PdCl₂, and 0.9 g of potassium carbonate, and then 10 mL of toluene and 1 mL of water were added as solvents. A reaction was conducted overnight at 100 °C under the protection of argon. After the reaction was completed, ethyl acetate and a saturated sodium chloride solution were added for extraction. The resultant organic layer was dried, filtered, concentrated, and purified by column chromatography to give 430 mg of the product.

### Step 11:

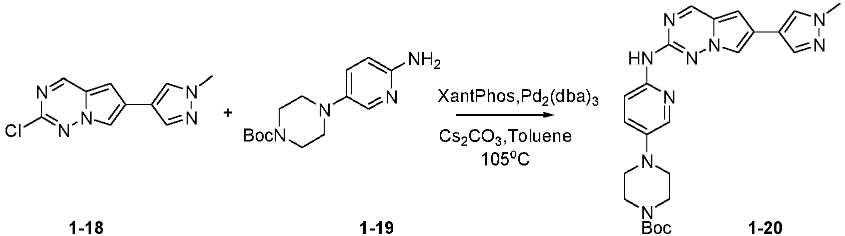

To a 100 mL flask were added 430 mg of Compound **1-18,** 565 mg of Compound **1-19,** 106 mg of XantPhos, 84 mg of Pd₂(dba)₃, and 900 mg of sodium tert-butoxide, and then 20 mL of toluene was added as a solvent. The mixture was heated to 105 °C and reacted overnight under the protection of argon. After the reaction was completed, a saturated sodium chloride solution was added, and the mixture was extracted with ethyl acetate. The resultant organic layer was dried, filtered, concentrated, and purified by column chromatography to give 200 mg of the product.

### Step 12:

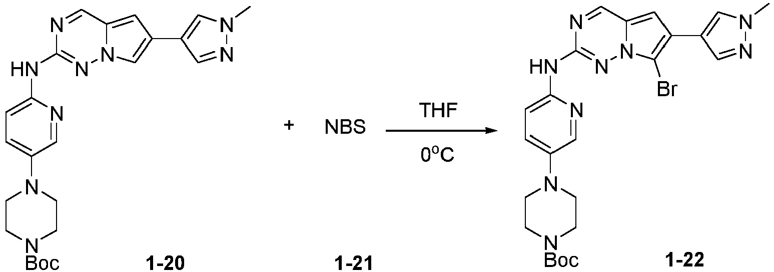

To a 50 mL flask was added 150 mg of Compound **1-20,** and then 15 mL of tetrahydrofuran was added as a solvent. After stirring at 0 °C for 10 min, 56 mg of NBS was slowly added and reacted for 15 min while stirring at 0 °C. After completion of the reaction was monitored, a saturated sodium chloride solution was added, and the mixture was extracted with ethyl acetate. The resultant organic layer was dried, filtered, concentrated, and purified by column chromatography to give 160 mg of the product.

### Step 13:

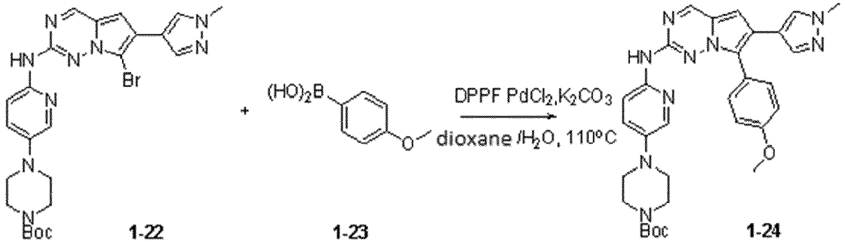

To a 50 mL flask were added 100 mg of Compound **1-22,** 42 mg of Compound **1-23,** 13 mg of DPPF PdCl₂, and 38 mg of potassium carbonate. Then, 15 mL of dioxane was added as a solvent, and 1.5 mL of water was added. The mixture was heated to 110 °C and reacted overnight under the protection of argon. After the reaction was completed, a saturated sodium chloride solution was added, and the mixture was extracted with ethyl acetate. The resultant organic layer was dried, filtered, concentrated, and purified by thin layer chromatography on a thick preparative plate to give 10 mg of the product.

### Step 14:

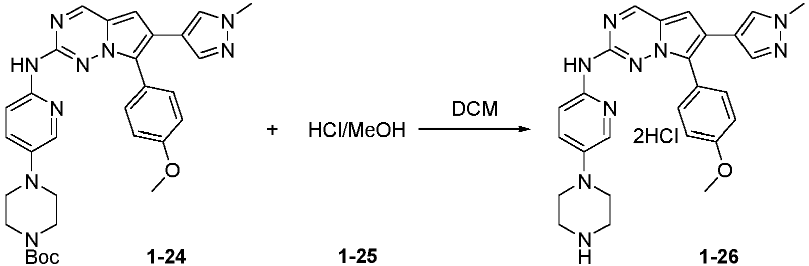

To a 25 mL flask was added 10 mg of Compound **1-24.** 5 mL of dichloromethane was added for dissolution, and then 0.5 mL of an HCl/MeOH solution (3 N) was added. The mixture was stirred and reacted for 2 h at room temperature. After the reaction was completed, the reaction mixture was concentrated to remove the solvent and excess HCl/MeOH, dried *in vacuo* to afford 9 mg of the product.

### Synthesis Scheme II

### Step 1:

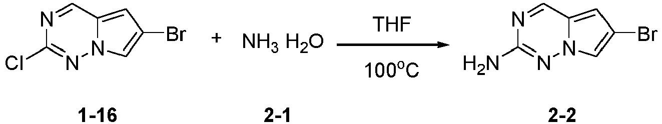

To a 50 mL pressure-resistant tube was added 2.2 g of Compound **1-16.** 10 mL of tetrahydrofuran was added as a solvent, and then 5 mL of aqueous ammonia (at a mass fraction of 28%) was added. The mixture was heated to 100 °C and reacted overnight. After the reaction was completed, the reaction mixture was concentrated directly to remove the solvent and excess aqueous ammonia to give 2.3 g of the product.

### Step 2:

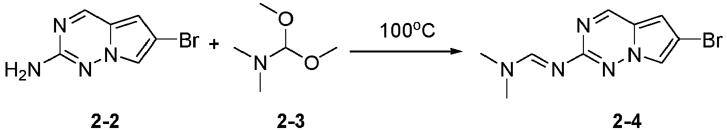

To a 50 mL flask were added 2.3 g of Compound **2-2** and 15 mL of Compound **2-3.** The mixture was heated to 100 °C and reacted for 4 h. After the reaction was completed, a saturated sodium chloride solution was added, and the mixture was extracted with ethyl acetate. The resultant organic layer was dried, filtered, and concentrated to give a crude product, which was washed with a mixture of methyl tert-butyl ether and n-hexane (1:1), and dried *in vacuo* to give 2.2 g of the product.

### Step 3:

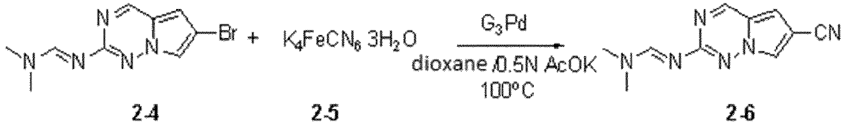

To a 10 mL flask were added 200 mg of Compound **2-4,** 211 mg of Compound **2-5,** 40 mg of a catalyst (CAS: 1447963-75-8), and 2.5 mL of a potassium acetate solution (0.5 N). The mixture was heated to 100 °C and reacted for 2 h under the protection of argon. After the reaction was completed, a saturated sodium chloride solution was added, and the mixture was extracted with ethyl acetate. The resultant organic layer was dried over anhydrous sodium sulfate, filtered, concentrated, and purified by column chromatography to give 110 mg of the product.

### Step 4:

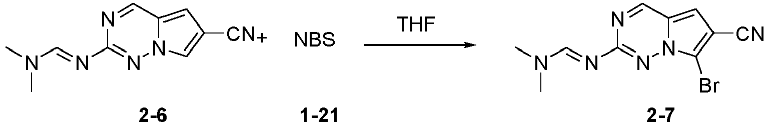

To a 50 mL flask were added 110 mg of Compound **2-6** and 20 mL of tetrahydrofuran. The mixture was stirred for 10 min in an ice-water bath. Then, 86 mg of Compound **21** was slowly added. After completion of the reaction was monitored, a saturated sodium chloride solution was added, and the mixture was extracted with ethyl acetate. The resultant organic layer was dried over anhydrous sodium sulfate, filtered, concentrated, and purified by column chromatography to give 150 mg of the product.

### Step 5:

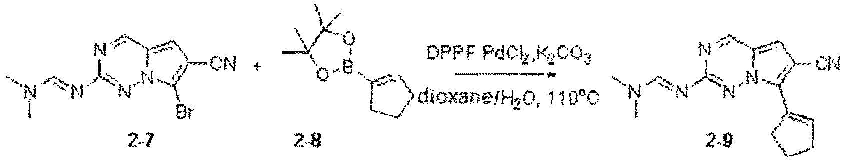

To a 50 mL flask were added 150 mg of Compound **2-7**, 149 mg of Compound **2-8**, 37.4 mg of DPPF PdCl₂, and 106 mg of potassium carbonate. Then, 15 mL of dioxane was added as a solvent, and 1.5 mL of water was added. The mixture was heated to 110 °C and reacted overnight under the protection of argon. After the reaction was completed, a saturated sodium chloride solution was added, and the mixture was extracted with ethyl acetate. The resultant organic layer was dried, filtered, concentrated, and purified by column chromatography to give 80 mg of the product.

### Step 6:

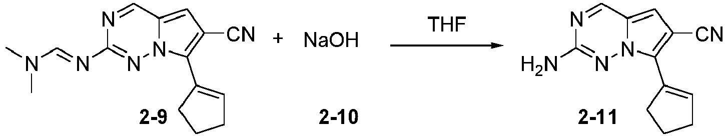

To a 50 mL flask was added 80 mg of Compound **2-9.** 10 mL of tetrahydrofuran was added as a solvent, and then 5 mL of a sodium hydroxide solution (2 N) was added. The mixture was heated to reflux and reacted for 4 h. After the reaction was completed, a saturated sodium chloride solution was added, and the mixture was extracted with ethyl acetate. The resultant organic layer was dried over anhydrous sodium sulfate, filtered, concentrated, and dried *in vacuo* to give 50 mg of the product.

### Step 7:

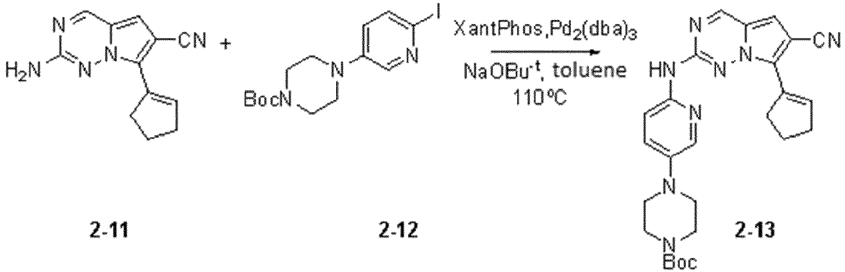

To a 50 mL flask were added 50 mg of Compound **2-11,** 129 mg of Compound **2-12,** 12.8 mg of XantPhos, 10 mg of Pd₂(dba)₃, and 32 mg of sodium tert-butoxide, and then 15 mL of toluene was added as a solvent. The mixture was heated to 110 °C and reacted overnight under the protection of argon. After the reaction was completed, a saturated sodium chloride solution was added, and the mixture was extracted with ethyl acetate. The resultant organic layer was dried over anhydrous sodium sulfate, filtered, concentrated, and purified by thin layer chromatography on a thick preparative plate to give 10 mg of the product.

### Step 8:

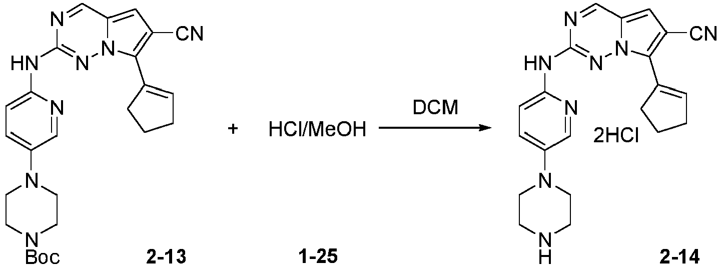

To a 25 mL flask was added 10 mg of Compound **2-13.** 5 mL of dichloromethane was added for dissolution, and then 0.5 mL of a HCl/MeOH solution (3 N) was added. The mixture was stirred and reacted for 2 h at room temperature. After the reaction was completed, the reaction mixture was concentrated to remove the solvent and excess HCl/MeOH, and dried *in vacuo* to afford 9 mg of the product.

### Synthesis Scheme III

### Step 1:

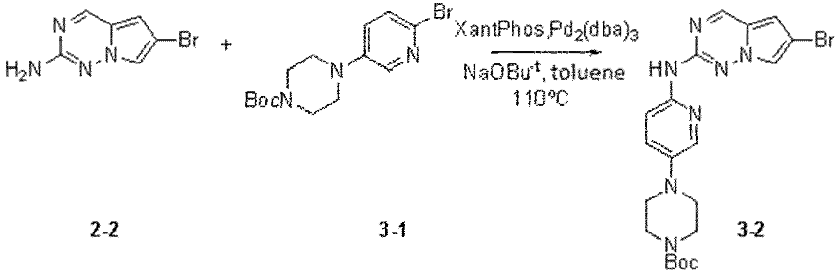

To a 100 mL flask were added 500 mg of Compound **2-2,** 964 mg of Compound **3-1,** 135.8 mg of XantPhos, 107.5 mg of Pd₂(dba)₃, and 338 mg of sodium tert-butoxide, and then 40 mL of toluene was added as a solvent. The mixture was heated to 105 °C and reacted overnight under the protection of argon. After the reaction was completed, a saturated sodium chloride solution was added, and the mixture was extracted with ethyl acetate. The resultant organic layer was dried over anhydrous sodium sulfate, filtered, concentrated, and purified by column chromatography to give 480 mg of the product.

### Step 2:

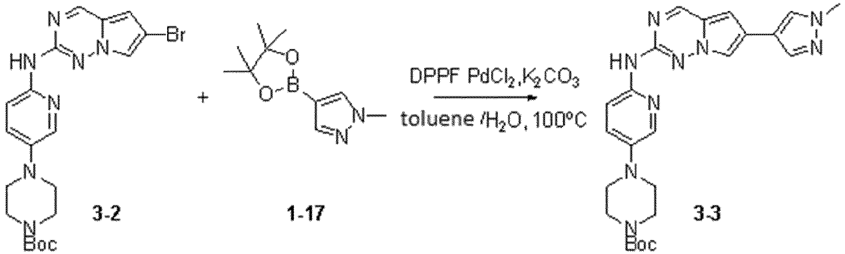

To a 50 mL flask were added 230 mg of Compound **3-2,** 121 mg of Compound **1-17,** 35 mg of DPPF PdCl₂, and 101 mg of potassium carbonate. 15 mL of dioxane was added as a solvent, and then 1.5 mL of water was added. The mixture was heated to 110 °C and reacted overnight under the protection of argon. After the reaction was completed, a saturated sodium chloride solution was added, and the mixture was extracted with ethyl acetate. The resultant organic layer was dried over anhydrous sodium sulfate, filtered, concentrated, and purified by column chromatography to give 150 mg of the product.

For other steps, please see Steps **12, 13,** and **14** in Synthesis Scheme **I.**

### Synthesis Scheme IV

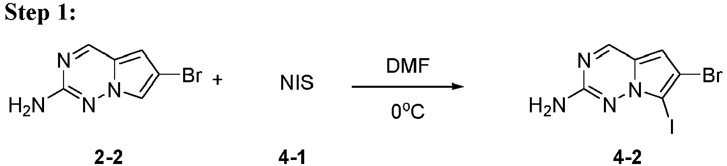

To a 100 mL flask was added 1 g of Compound **2-2,** and then 50 mL of DMF was added for dissolution. The mixture was stirred for 10 min in an ice-water bath, and then 1.06 g of Compound **4-1** was added (in three portions, 15 min apart). After a 0.5 h reaction, the reaction was monitored. After the reaction was completed, a saturated sodium chloride solution was added, and the mixture was extracted with ethyl acetate. The resultant organic layer was dried over anhydrous sodium sulfate, filtered, concentrated, and purified by column chromatography to give 0.9 g of the product.

### Step 2:

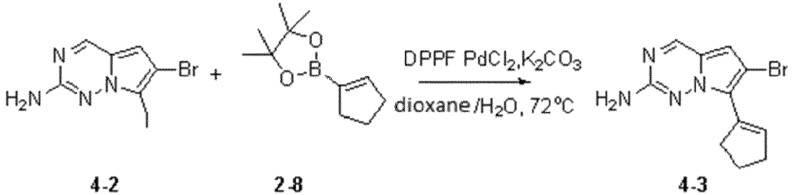

To a 100 mL flask were added 0.9 g of Compound **4-2,** 610 mg of Compound **2-8,** 194 mg of DPPF PdCl₂, and 732 mg of potassium carbonate. 50 mL of dioxane was added as a solvent, and then 5 mL of water was added. The mixture was heated to 72 °C and reacted overnight under the protection of argon. After the reaction was completed, a saturated sodium chloride solution was added, and the mixture was extracted with ethyl acetate. The resultant organic layer was dried, filtered, concentrated, and purified by column chromatography to give 380 mg of the product.

### Step 3:

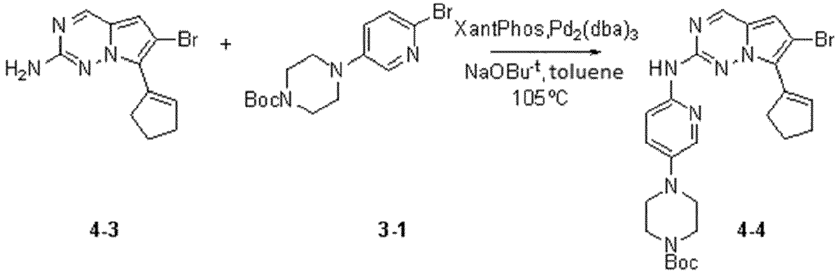

To a 100 mL flask were added 380 mg of Compound **4-3,** 559 mg of Compound **3-1,** 78 mg of XantPhos, 62 mg of Pd₂(dba)₃, and 131 mg of sodium tert-butoxide, and then 30 mL of toluene was added as a solvent. The mixture was heated to 105°C and reacted overnight under the protection of argon. After the reaction was completed, a saturated sodium chloride solution was added, and the mixture was extracted with ethyl acetate. The resultant organic layer was dried over anhydrous sodium sulfate, filtered, concentrated, and purified by column chromatography to give 390 mg of the product.

### Step 4:

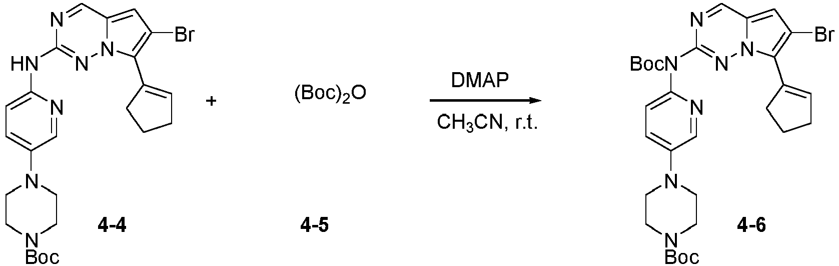

To a 100 mL flask were added 43 mg of Compound **4-4**, 52 mg of Compound **4-5,** and 1 mg of DMAP, and then acetonitrile was added as a solvent. A reaction was conducted overnight at room temperature. After the reaction was completed, a saturated sodium chloride solution was added, and the mixture was extracted with ethyl acetate. The resultant organic layer was dried over anhydrous sodium sulfate, filtered, concentrated, and purified by column chromatography to give 35 mg of the product.

### Step 5:

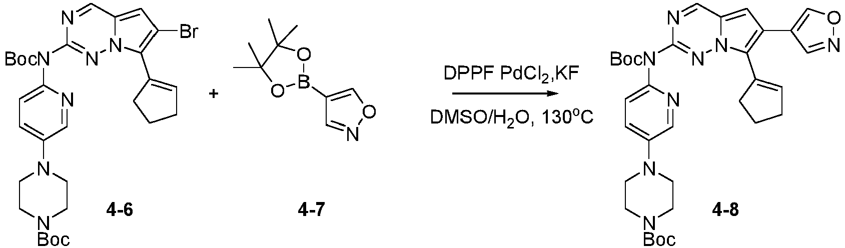

To a 100 mL flask were added 35 mg of Compound **4-6**, 21 mg of Compound **4-7**, 4 mg of DPPF PdCl₂, and 9.5 mg of potassium fluoride, and then 5 mL of DMSO was added as a solvent. The mixture was heated in microwave to 130 °C and reacted for 1 h under the protection of argon. After the reaction was completed, a saturated sodium chloride solution was added, and the mixture was extracted with ethyl acetate. The resultant organic layer was dried over anhydrous sodium sulfate, filtered, concentrated, and purified by thin layer chromatography to give 7 mg of the product.

### Step 6:

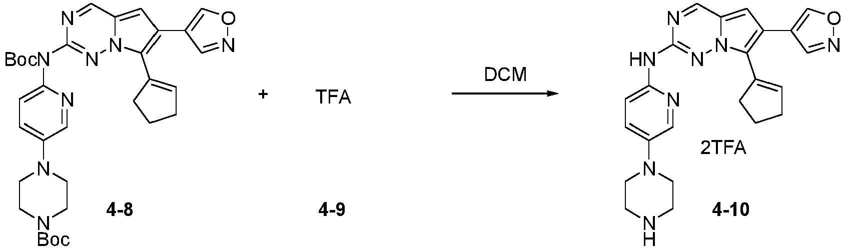

To a 25 mL flask was added 7 mg of Compound **4-8.** 5 mL of dichloromethane was added for dissolution, and then 0.5 mL of TFA was added. A reaction was conducted for 2 h while stirring at room temperature. After the reaction was completed, the reaction mixture was concentrated to remove the solvent and excess TFA, and dried *in vacuo* to afford 7 mg of the product.

### Synthesis Scheme V

### Step 1:

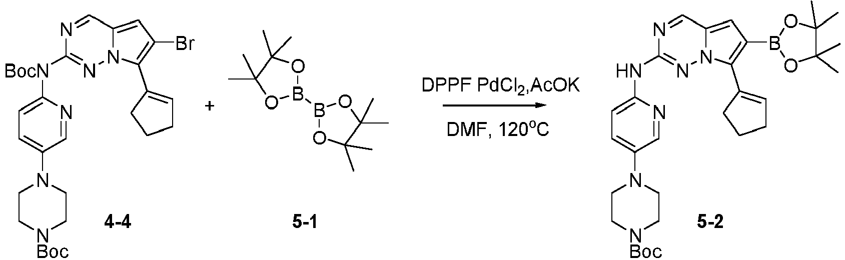

To a microwave tube were added Compound **4-4** (1.0 g), Bis(pinacolato)diboron (1.19 g), PdCl₂(dppf) (57.11 mg), potassium acetate (459.63 mg), and DMF (15 mL). A reaction was conducted at 135 °C in microwave for 1.5 h under the protection of argon. After the reaction was completed, EA and H₂O were added for extraction, and layers were separated. The organic phase was dried to remove water, spin-dried, and purified through a column (gradient elution, eluents: EA:Hex = 1:5 ∼ 1:1) to give Compound **5-2** (787.6 mg).

### Step 2:

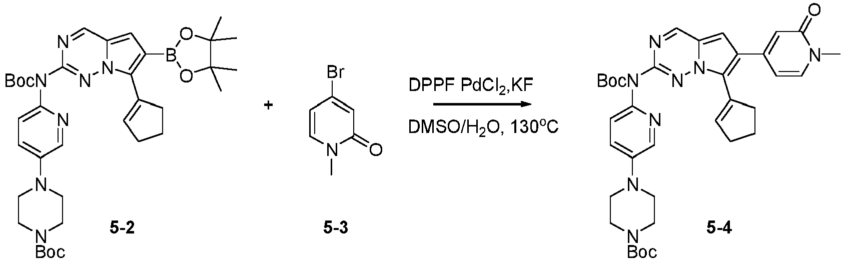

To a microwave tube were added Compound **5-2** (50 mg), Compound **5-3** (34.18 mg), PdCl₂(dppf) (5.32 mg), potassium fluoride (21.12 mg), DMSO (5 mL), and water (0.5 mL). A reaction was conducted at 130 °C in microwave for 1.5 h under the protection of argon. After the reaction was completed, EA and H₂O were added for extraction, and layers were separated. The organic phase was spin-dried and purified by thin layer chromatography to give Compound **5-4** (7 mg).

### Step 3:

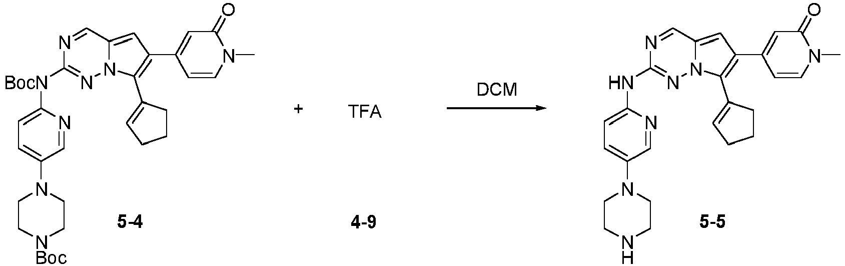

To a 25 mL flask was added 7 mg of Compound **5-4.** 5 mL of dichloromethane was added for dissolution, and then 0.5 mL of TFA was added. The mixture was stirred and reacted at room temperature for 2 h. After the reaction was completed, the reaction mixture was concentrated to remove the solvent and excess TFA, and dried *in vacuo* to afford 6.3 mg of the product.

### Synthesis Scheme VI

### Step 1:

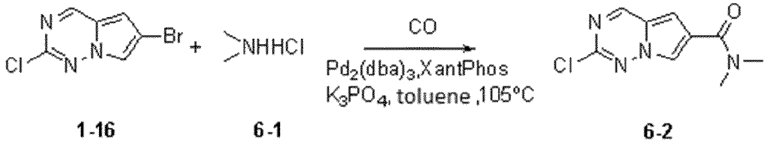

To a 100 mL reaction flask were added Compound **1-16** (500 mg), dimethylamine hydrochloride (210.47 mg), Pd₂(dba)₃ (147.72 mg), Xantphos (186.68 mg), K₃PO₄ (1.0 g), and toluene (15 mL). CO gas was charged, and the mixture was heated to 105 °C and stirred overnight. The reaction was suction filtered through a Celite pad, and rinsed with DCM. The filtrate was spin-dried and purified through a column (gradient elution, eluents: EA:Hex = 1:3~3:1) to give the product **6-2** (250 mg).

### Step 2:

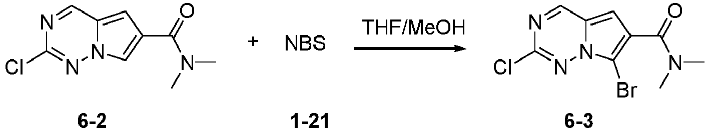

To a reaction flask were added Compound **6-2** (250 mg), THF (9 mL), and MeOH (3 mL) in an ice-water bath, and NBS (200 mg) was added in portions. The mixture was stirred for 1 h. After the ice bath was removed and the temperature was raised to room temperature, the mixture was stirred for another 1 h. After the reaction was completed, EA and H₂O were added, set aside, and layers were separated. The organic phase was dried, spin-dried, and purified through a column (gradient elution, eluents: EA:Hex = 1:1∼3:1) to give Compound **6-3** (125 mg).

### Step 3:

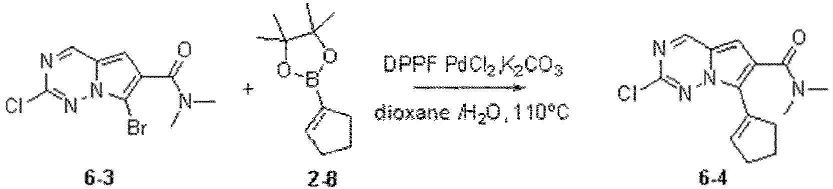

To a microwave tube were added Compound **6-3** (150 mg), 1-cyclopentene boronic acid pinacol (143.86 mg), the catalyst PdCl₂(dppf) (36.16 mg), and potassium carbonate (170.74 mg), and then dioxane (4 mL) and water (0.4 mL) were added as solvents. A reaction was conducted at 110 °C in microwave for 1 h under the protection of argon. After the reaction was completed, EA and H₂O were added, set aside, and layers were separated. The organic phase was dried over anhydrous sodium sulfate, spin-dried, and purified through a column (gradient elution, eluents: EA:Hex = 1:1∼3:1) to give Compound **6-4** (100 mg).

### Step 4:

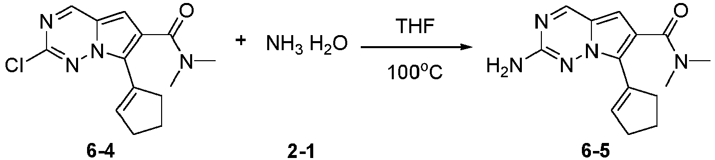

To a 20 mL pressure-resistant tube was added 450 mg of Compound **6-4.** Tetrahydrofuran (4 mL) was added as a solvent, and then 8 mL of aqueous ammonia (at a mass fraction of 28%) was added. The mixture was heated to 100 °C and reacted overnight. After the reaction was completed, the reaction mixture was directly concentrated to remove the solvent and excess aqueous ammonia to give 300 mg of the product.

### Step 5:

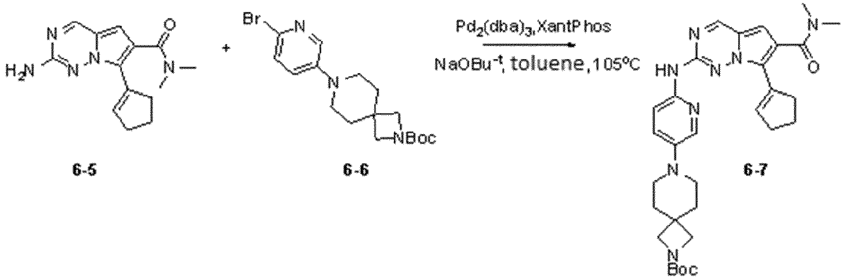

To a reaction flask were added Compound **6-5** (40 mg), Compound **6-6** (67.63 mg), Pd₂(dba)₃ (6.75 mg), Xantphos (9.38 mg), sodium tert-butoxide (35.42 mg), and toluene (10 mL). The mixture was heated to 105 °C and stirred overnight under the protection of argon. After the reaction was completed, solids were filtered off, and the filtrate was spin-dried and purified by thin layer chromatography to give Compound **6-7** (43 mg).

### Step 6:

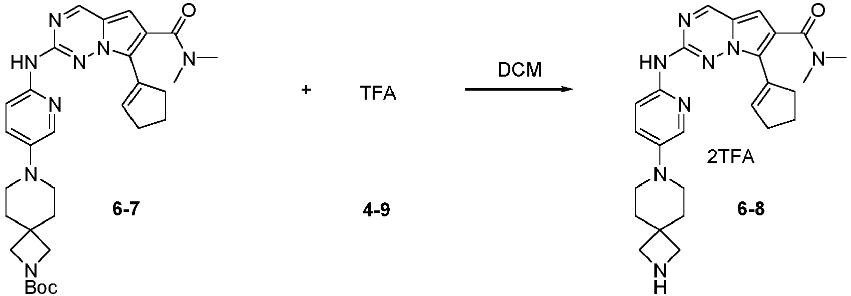

To a 25 mL flask was added 43 mg of Compound **6-7.** 5 mL of dichloromethane was added for dissolution, and then 1 mL of TFA was added. The mixture was stirred and reacted at room temperature for 2 h. After the reaction was completed, the reaction mixture was concentrated to remove the solvent and excess TFA, and dried *in vacuo* to afford 60 mg of the product.

### Synthesis Scheme VII

### Step 1:

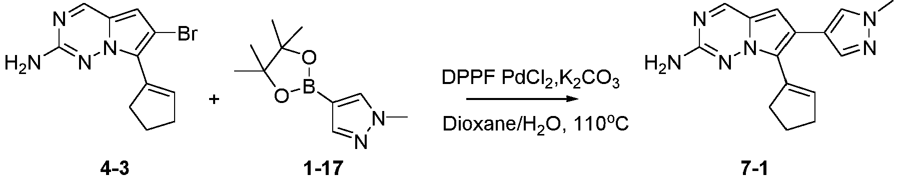

To a 50 mL flask were added 150 mg of Compound **4-3**, 168 mg of Compound **1-17,** 39 mg of DPPF PdCl₂, and 148 mg of potassium carbonate, and then 10 mL of toluene and 1 mL of water were added as solvents. A reaction was conducted overnight at 110 °C under the protection of argon. After the reaction was completed, ethyl acetate and a saturated sodium chloride solution were added for extraction. The resultant organic layer was dried, filtered, concentrated, and purified by column chromatography to give 90 mg of the product.

### Step 2:

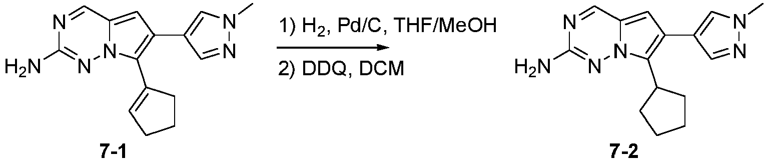

To a 100 mL reaction flask were added 90 mg of Compound **7-1,** an appropriate amount of Pd/C catalyst, followed by 8 mL of tetrahydrofuran and 15 mL of methanol. The mixture was purged with hydrogen, and reacted for 2 h at room temperature under a hydrogen atmosphere. After the reaction was completed, the reaction mixture was filtered, and the filtrate was concentrated and dried *in vacuo* to give an intermediate product.

The obtained intermediate product was dissolved in 15 mL of dichloromethane, and then 100 mg of DDQ was added. The mixture was stirred and reacted at room temperature for 1 h. After the reaction was completed, the reaction mixture was filtered. A saturated sodium bicarbonate solution was added to the filtrate, which was extract with ethyl acetate to give a organic layer. The organic layer was dried, concentrated, and purified by column chromatography to give 50 mg of the product **(7-2).**

### Step 3:

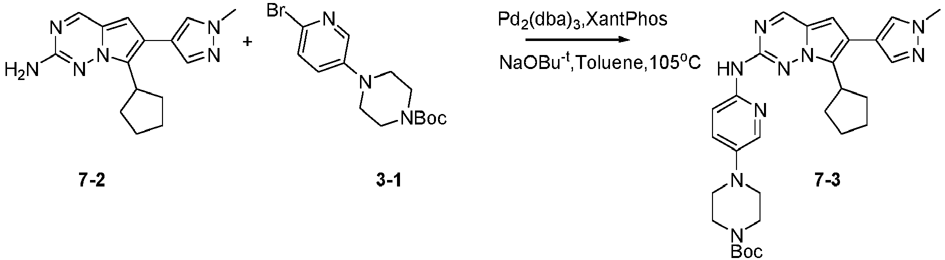

To a reaction flask were added 20 mg of Compound **7-2**, 37 mg of Compound **3-1**, 4 mg of Pd₂(dba)₃, 5 mg of Xantphos, 14 mg of sodium tert-butoxide, and 10 mL of toluene. Under the protection of argon, the mixture was heated to 105°C and stirred overnight. After the reaction was completed, solid was filtered off, and the filtrate was spin-dried and purified by thin layer chromatography to give 7 mg of Compound **7-3.**

### Step 4:

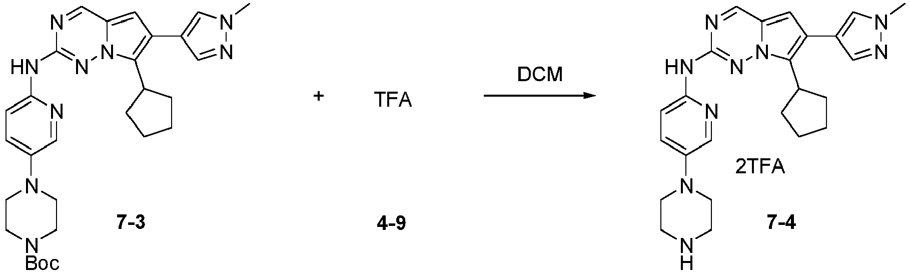

To a 25 mL flask was added 7 mg of Compound **7-3**. 5 mL of dichloromethane was added for dissolution, and then 1 mL of TFA was added. The mixture was stirred and reacted at room temperature for 2 h. After the reaction was completed, the mixture was concentrated to remove the solvent and excess TFA, and dried *in vacuo* to afford 8.2 mg of the product **7-4.**

The salt-forming compounds obtained in the above Synthesis Schemes I to VII can be prepared into non-salt-forming forms by methods known in the art, for example, using the following Desalination Scheme I or II.

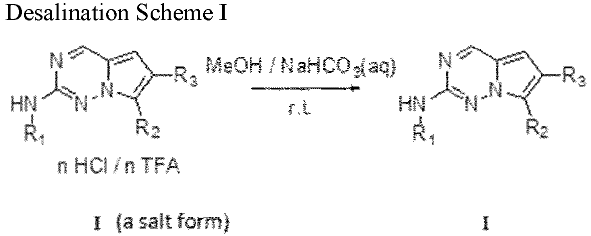

To a flask was added a salt form of Compound **I.** Methanol was added as a solvent, and then an excess amount of a saturated sodium bicarbonate solution was added. The mixture was stirred and reacted at room temperature for 4 h. After the reaction was completed, the reaction mixture was concentrated to remove part of methanol, and extracted with dichloromethane. The resultant organic layer was dried over anhydrous sodium sulfate, filtered, concentrated to remove the solvent, and dried *in vacuo* to afford the target compound **I.**

A more specific example (the compound of Example 111 as described below) is as follows:

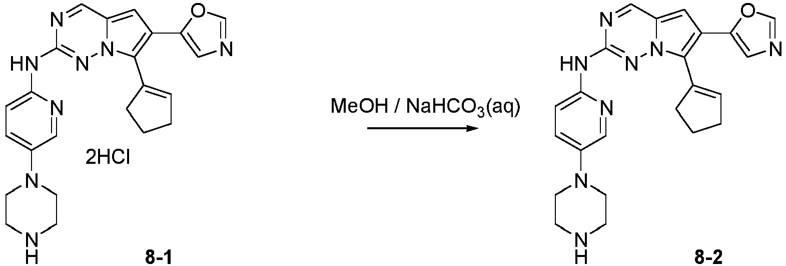

To a 25 mL flask was added 13 mg of Compound **8-1.** 3 mL of methanol was added as a solvent, and then 2 mL of a saturated sodium bicarbonate solution was added. The mixture was stirred and reacted at room temperature for 4 h. After the reaction was completed, the reaction mixture was concentrated to remove part of methanol, and extracted with dichloromethane. The resultant organic layer was dried over anhydrous sodium sulfate, filtered, concentrated to remove the solvent, and dried *in vacuo* to afford 9.4 mg of the target compound **8-2.**

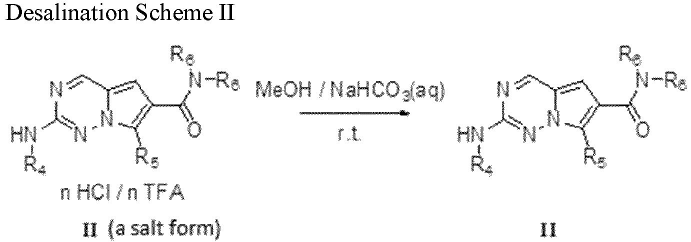

To a flask was added a salt form of Compound **II.** Methanol was added as a solvent, and then an excess amount of a saturated sodium bicarbonate solution was added. The mixture was stirred and reacted at room temperature for 4 h. After the reaction was completed, the reaction mixture was concentrated to remove part of methanol, and extracted with dichloromethane. The resultant organic layer was dried over anhydrous sodium sulfate, filtered, concentrated to remove the solvent, and dried *in vacuo* to afford the target compound **II.**

A more specific example (the compound of Example 112 as described below) is as follows:

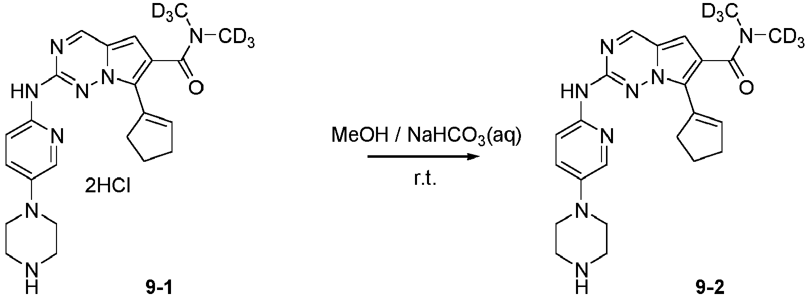

To a 25 mL flask was added 27 mg of Compound **9-1.** 3 mL of methanol was added as a solvent, and then 3 mL of a saturated sodium bicarbonate solution was added. The mixture was stirred and reacted at room temperature for 4 h. After the reaction was completed, the reaction mixture was concentrated to remove part of methanol, and extracted with dichloromethane. The resultant organic layer was dried over anhydrous sodium sulfate, filtered, concentrated to remove the solvent, and dried *in vacuo* to afford 20.5 mg of the target compound **9-2.**

### Test Example 1: Analysis of in vitro inhibitory activity on CDK kinase

In an *in vitro* cell-free kinase activity assay, the half inhibitory concentration (IC₅₀) of the compound of the present invention on CDK6/CyclinD1 (trade name: Carna, product No. 04-114) was detected by the method as below. It should be noted that the assay can also be conducted by a similar method. The assay steps in this Test Example are as follows:
The CDK6/CyclinD1 kinase activity was determined using a time-resolved fluorescence resonance energy transfer (TR-FRET) method. Measurements were performed in a reaction volume of 20 µL using 384-well assay plates. The kinase, inhibitor, ATP (at a concentration equivalent to the Kₘ for CDK6/CyclinD1 kinase, trade name: Sigma, product No. A7699), and polypeptide substrate (at a concentration equivalent to the Kₘ for CDK6/CyclinD1 kinase, trade name: Cisbio, product No. 64CUS000C01B) were incubated in a reaction buffer consisting of 20 mM HEPES (pH 7.5), 1 mM EGTA, 10 mM MgCl₂, 0.02% Briji35, 0.02 mg/ml BSA, 0.1 mM Na₃VO₄, 2 mM DTT, and 1% DMSO (pH 7.5) for 1.5 h. The reaction was stopped by adding a mixture of a peptide antibody (trade name: Cisbio, product No. 64CUSKAY) and Sa-XL665 (trade name: Cisbio, product No. 610SAXLB) diluted in HTRF KinEASE detection buffer (trade name: Cisbio, product No. 62SDBRDF, containing EDTA). After the mixture was incubated for 1 hour at room temperature, the plate was read. The TR-FRET signal was measured on a multimode plate reader (EnVision^{™}, a multi-function microplate detector, PerkinElmer) at an excitation wavelength (λ_{Ex}) of 330 nm and detection wavelengths (λ_{Em}) of 615 and 665 nm. The activity was determined by the ratio of fluorescence at 665 nm to that at 615 nm. For each compound, the enzyme activities of different concentrations of the compound were measured to determine IC₅₀. The negative control contained no inhibitor. Two no-enzyme controls were used to determine baseline fluorescence levels. IC₅₀ was obtained by fitting using the GraphPad 6.02 software.

Each Example compound and Reference Example compound were prepared following the schemes described above, as shown in Table 1 for details. 8-10 concentrations were prepared for each compound, and formulated in DMSO. The stock concentration was 200× the working concentration. For example, if the working concentration was 100 nM, the corresponding concentration of the formulated stock solution was 20 µM.

In the analysis of *in vitro* inhibitory activity on CDK kinase, the IC₅₀ value of each of the Example compounds and Reference Example compounds was determined. The IC₅₀ values are given according to the interval of the IC₅₀ value, where "+++" represents IC₅₀ < 100 nM; "++" represents 100 nM ≤ IC₅₀ < 1000 nM; "+" represents 1000 nM ≤ IC₅₀ < 3000 nM; and "-" represents "more than 3000 nM".

**Table 1. Example compounds and their IC₅₀ values against CDK6/CyclinD1 kinase**

| No. | Structure | Synthesis Scheme | Data | IC₅₀ (CDK6) |
|---|---|---|---|---|
| Example 1 | 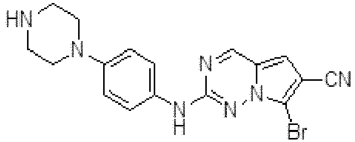 | Following Synthesis Scheme II | MS-ESI: 398(M+1) | +++ |
| Example 2 | 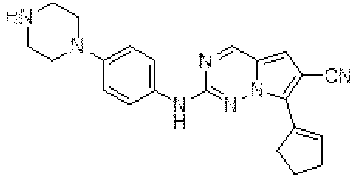 | Following Synthesis Scheme II | MS-ESI: 386(M+1) | +++ |
| Example 3 | 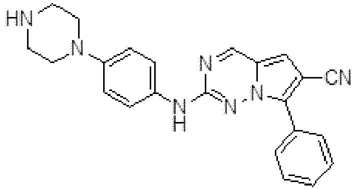 | Following Synthesis Scheme II | MS-ESI: 396(M+1) | +++ |
| Example 4 | 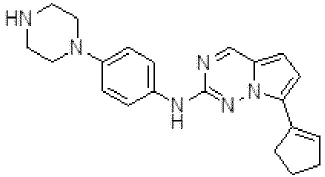 | Following Synthesis Scheme II | MS-ESI: 361(M+1) | ++ |
| Example 5 | 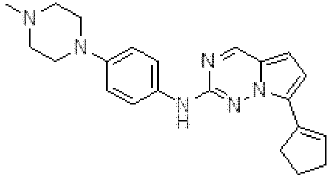 | Following Synthesis Scheme II | MS-ESI: 375(M+1) | ++ |
| Example 6 | 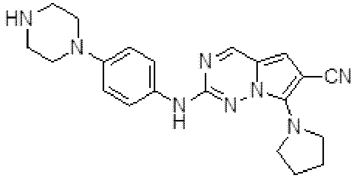 | Following Synthesis Scheme II | MS-ESI: 389(M+1) | +++ |
| Example 7 | 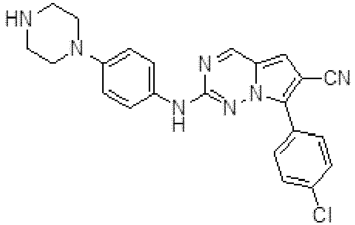 | Following Synthesis Scheme II | MS-ESI: 430(M+1) | +++ |
| Example 8 | 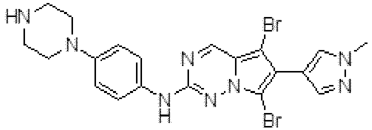 | Following Synthesis Scheme I | MS-ESI: 531(M+1) | +++ |
| Example 9 | 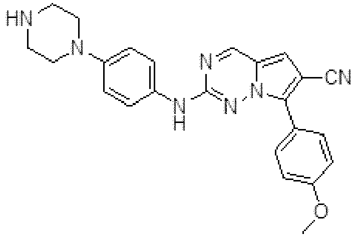 | Following Synthesis Scheme II | MS-ESI: 426(M+1) | +++ |
| Example 10 | 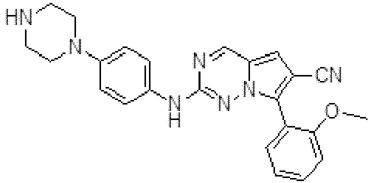 | Following Synthesis Scheme II | MS-ESI: 426(M+1) | +++ |
| Example 11 | 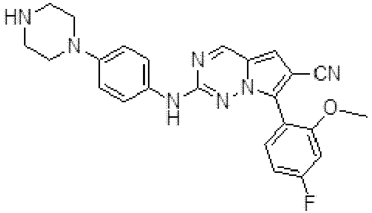 | Following Synthesis Scheme II | MS-ESI: 444(M+1) | +++ |
| Example 12 | 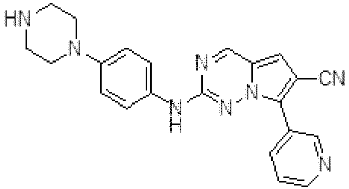 | Following Synthesis Scheme II | MS-ESI: 397(M+1) | +++ |
| Example 13 | 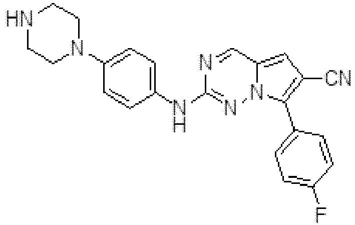 | Following Synthesis Scheme II | MS-ESI: 414(M+1) | +++ |
| Example 14 | 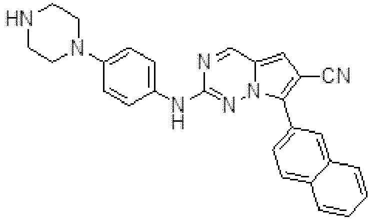 | Following Synthesis Scheme II | MS-ESI: 446(M+1) | +++ |
| Example 15 | 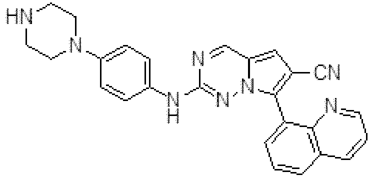 | Following Synthesis Scheme II | MS-ESI: 447(M+1) | ++ |
| Example 16 | 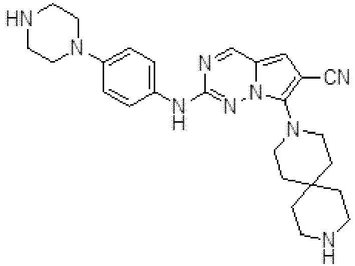 | Following Synthesis Scheme II | MS-ESI: 472(M+1) | +++ |
| Example 17 | 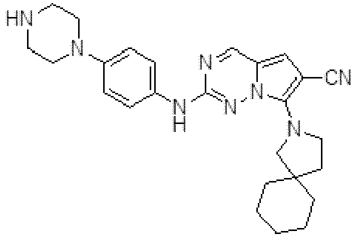 | Following Synthesis Scheme II | MS-ESI: 457(M+1) | +++ |
| Example 18 | 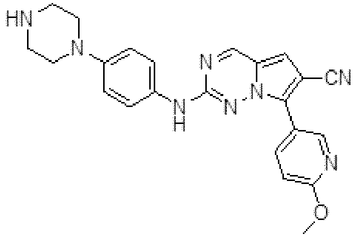 | Following Synthesis Scheme II | MS-ESI: 427(M+1) | +++ |
| Example 19 | 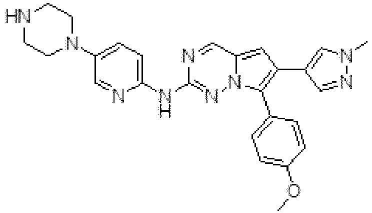 | Following Synthesis Scheme I or III | MS-ESI: 482(M+1) | +++ |
| Example 20 | 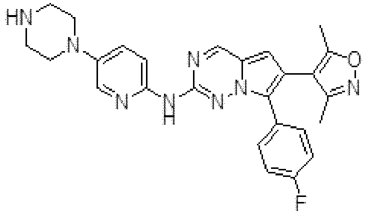 | Following Synthesis Scheme I or III | MS-ESI: 485(M+1) | ++ |
| Example 21 | 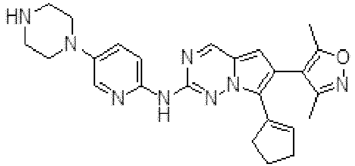 | Following Synthesis Scheme I or III | MS-ESI: 457(M+1) | ++ |
| Example 22 | 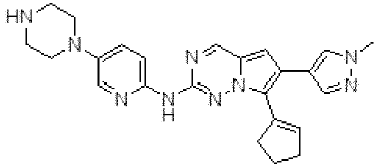 | Following Synthesis Scheme I or III | MS-ESI: 442(M+1) | +++ |
| Example 23 | 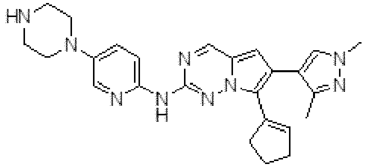 | Following Synthesis Scheme I or III | MS-ESI: 456(M+1) | ++ |
| Example 24 | 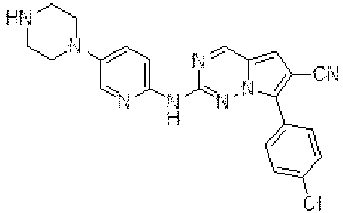 | Following Synthesis Scheme II | MS-ESI: 431(M+1) | +++ |
| Example 25 | 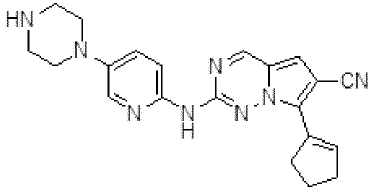 | Following Synthesis Scheme II | MS-ESI: 387(M+1) | +++ |
| Example 26 | 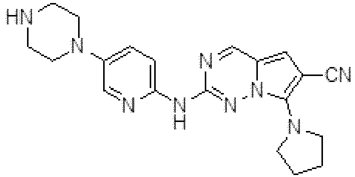 | Following Synthesis Scheme II | MS-ESI: 390(M+1) | +++ |
| Example 27 | 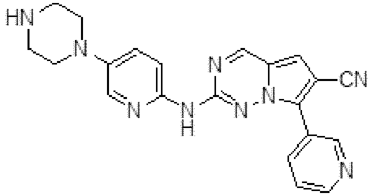 | Following Synthesis Scheme II | MS-ESI: 398(M+1) | ++ |
| Example 28 | 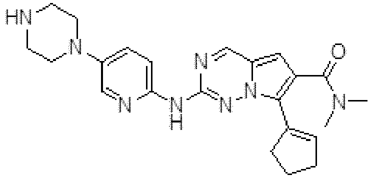 | Following Synthesis Scheme VI | MS-ESI: 433(M+1) | +++ |
| Example 29 | 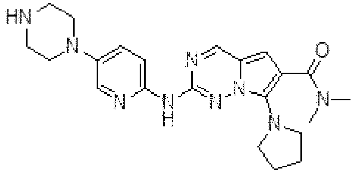 | Following Synthesis Scheme VI | MS-ESI: 436(M+1) | +++ |
| Example 30 | 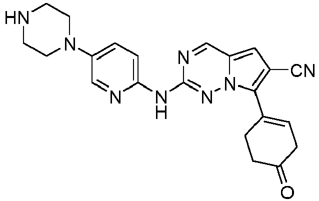 | Following Synthesis Scheme II | MS-ESI: 415(M+1) | ++ |
| Example 31 | 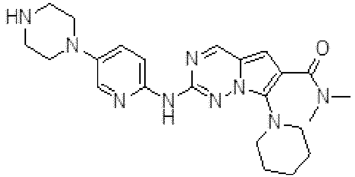 | Following Synthesis Scheme VI | MS-ESI: 450(M+1) | +++ |
| Example 32 | 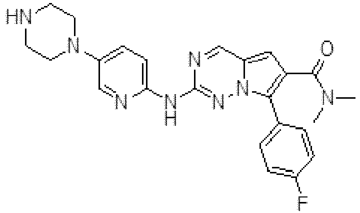 | Following Synthesis Scheme VI | MS-ESI: 461(M+1) | +++ |
| Example 33 | 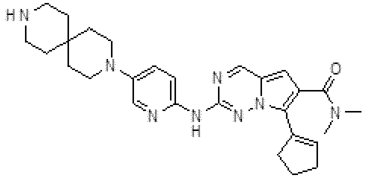 | Following Synthesis Scheme VI | MS-ESI: 501(M+1) | +++ |
| Example 34 | 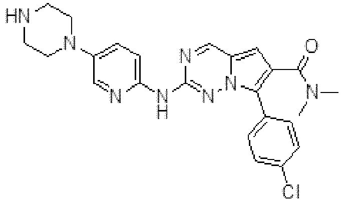 | Following Synthesis Scheme VI | MS-ESI: 477(M+1) | +++ |
| Example 35 | 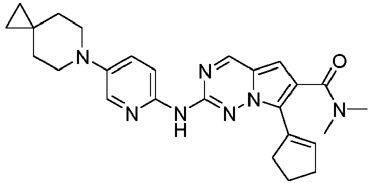 | Following Synthesis Scheme VI | MS-ESI: 458(M+1) | ++ |
| Example 36 | 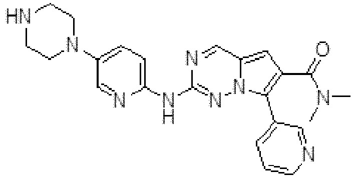 | Following Synthesis Scheme VI | MS-ESI: 444(M+1) | ++ |
| Example 37 | 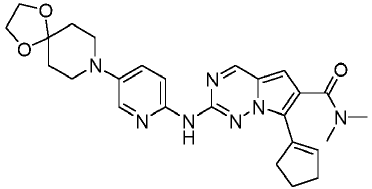 | Following Synthesis Scheme VI | MS-ESI: 490(M+1) | +++ |
| Example 38 | 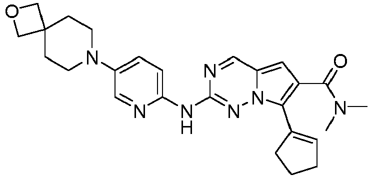 | Following Synthesis Scheme VI | MS-ESI: 474(M+1) | +++ |
| Example 39 | 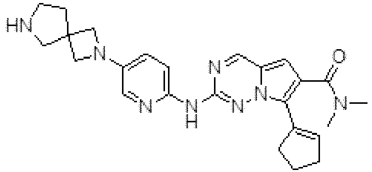 | Following Synthesis Scheme VI | MS-ESI: 459(M+1) | +++ |
| Example 40 | 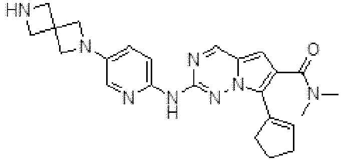 | Following Synthesis Scheme VI | MS-ESI: 445(M+1) | +++ |
| Example 41 | 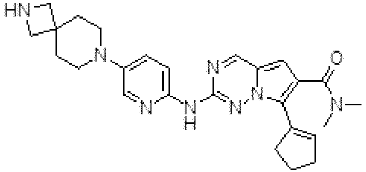 | Following Synthesis Scheme VI | MS-ESI: 473(M+1) | +++ |
| Example 42 | 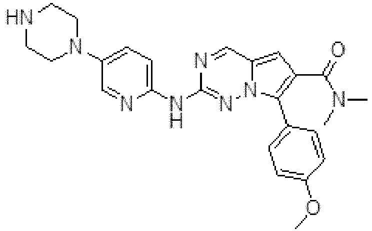 | Following Synthesis Scheme VI | MS-ESI: 473(M+1) | +++ |
| Example 43 | 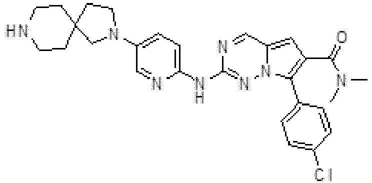 | Following Synthesis Scheme VI | MS-ESI: 531(M+1) | +++ |
| Example 44 | 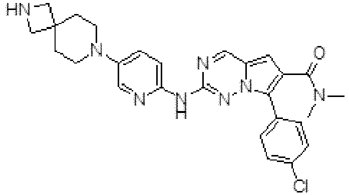 | Following Synthesis Scheme VI | MS-ESI: 517(M+1) | +++ |
| Example 45 | 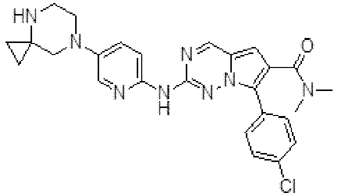 | Following Synthesis Scheme VI | MS-ESI: 503(M+1) | +++ |
| Example 46 | 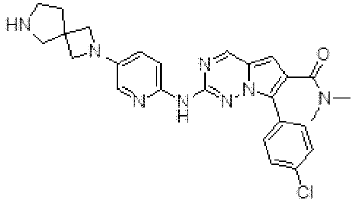 | Following Synthesis Scheme VI | MS-ESI: 503(M+1) | +++ |
| Example 47 | 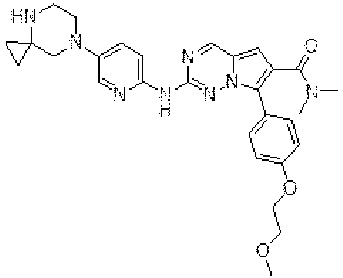 | Following Synthesis Scheme VI | MS-ESI: 543(M+1) | +++ |
| Example 48 | 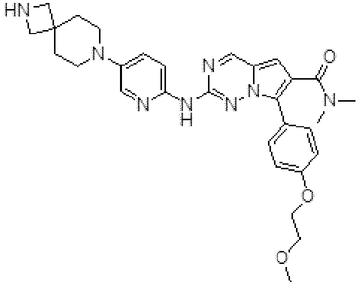 | Following Synthesis Scheme VI | MS-ESI: 557(M+1) | +++ |
| Example 49 | 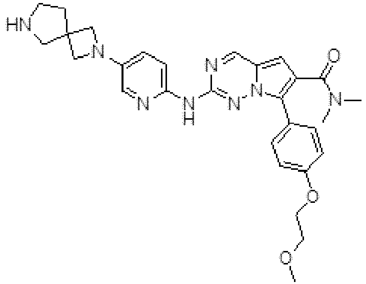 | Following Synthesis Scheme VI | MS-ESI: 543(M+1) | +++ |
| Example 50 | 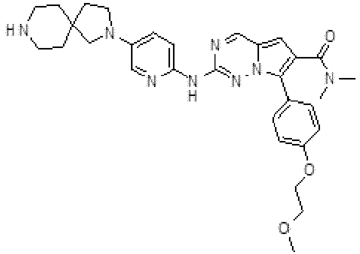 | Following Synthesis Scheme VI | MS-ESI: 571(M+1) | +++ |
| Example 51 | 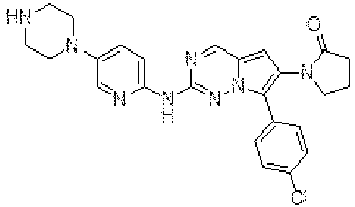 | Following Synthesis Scheme III | MS-ESI: 489(M+1) | ++ |
| Example 52 | 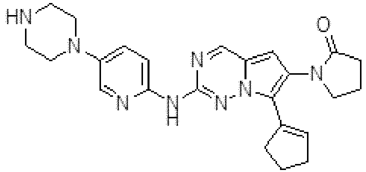 | Following Synthesis Scheme III | MS-ESI: 445(M+1) | ++ |
| Example 53 | 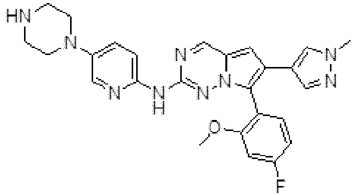 | Following Synthesis Scheme III | MS-ESI: 500(M+1) | ++ |
| Example 54 | 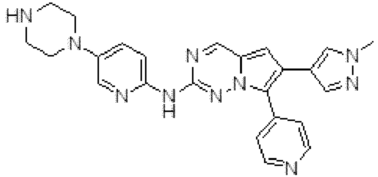 | Following Synthesis Scheme III | MS-ESI: 453(M+1) | +++ |
| Example 55 | 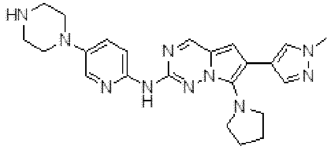 | Following Synthesis Scheme III | MS-ESI: 445(M+1) | +++ |
| Example 56 | 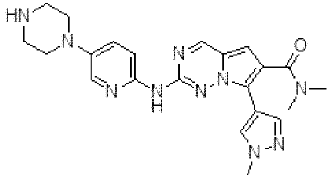 | Following Synthesis Scheme VI | MS-ESI: 447(M+1) | ++ |
| Example 57 | 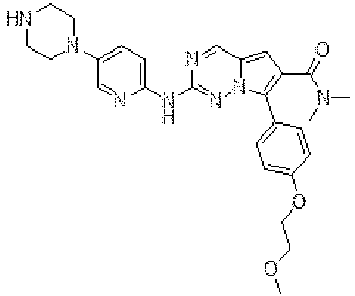 | Following Synthesis Scheme VI | MS-ESI: 517(M+1) | +++ |
| Example 58 | 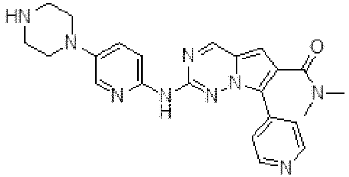 | Following Synthesis Scheme VI | MS-ESI: 444(M+1) | +++ |
| Example 59 | 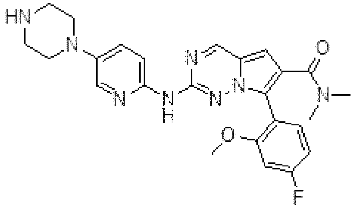 | Following Synthesis Scheme VI | MS-ESI: 491(M+1) | +++ |
| Example 60 | 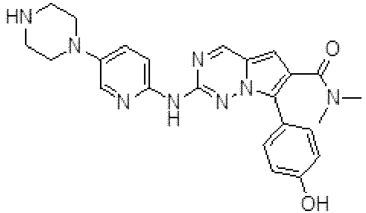 | Following Synthesis Scheme VI | MS-ESI: 459(M+1) | +++ |
| Example 61 | 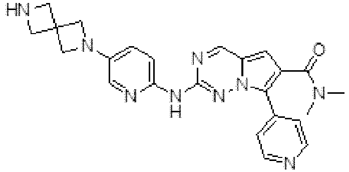 | Following Synthesis Scheme VI | MS-ESI: 456(M+1) | +++ |
| Example 62 | 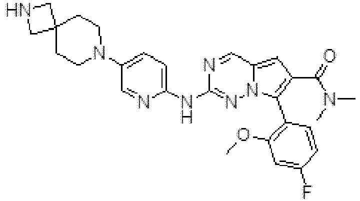 | Following Synthesis Scheme VI | MS-ESI: 531(M+1) | ++ |
| Example 63 | 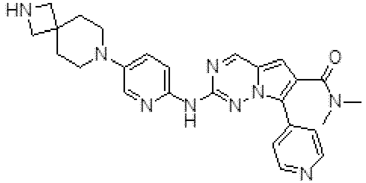 | Following Synthesis Scheme VI | MS-ESI: 484(M+1) | +++ |
| Example 64 | 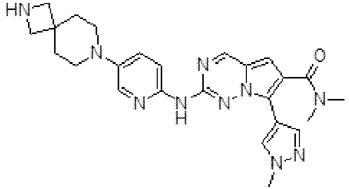 | Following Synthesis Scheme VI | MS-ESI: 487(M+1) | ++ |
| Example 65 | 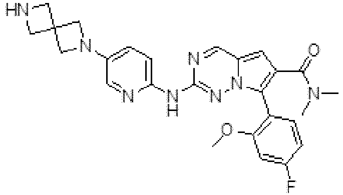 | Following Synthesis Scheme VI | MS-ESI: 503(M+1) | +++ |
| Example 66 | 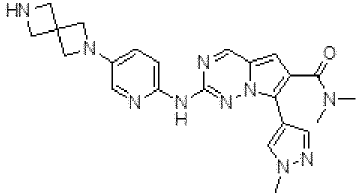 | Following Synthesis Scheme VI | MS-ESI: 459(M+1) | +++ |
| Example 67 | 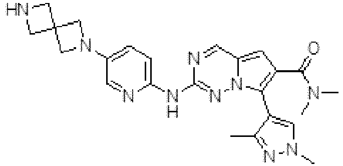 | Following Synthesis Scheme VI | MS-ESI: 473(M+1) | ++ |
| Example 68 | 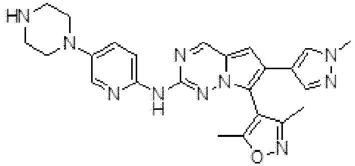 | Following Synthesis Scheme III | MS-ESI: 471(M+1) | ++ |
| Example 69 | 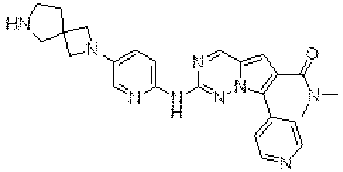 | Following Synthesis Scheme VI | MS-ESI: 470(M+1) | +++ |
| Example 70 | 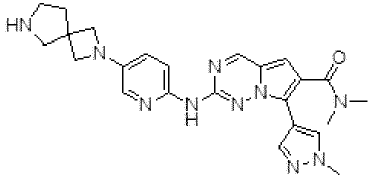 | Following Synthesis Scheme VI | MS-ESI: 473(M+1) | +++ |
| Example 71 | 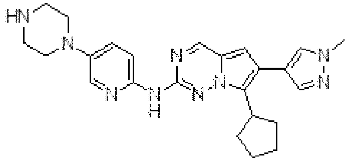 | Following Synthesis Scheme VII | MS-ESI: 444(M+1) | +++ |
| Example 72 | 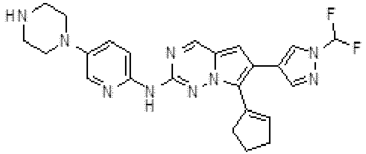 | Following Synthesis Scheme IV | MS-ESI: 478(M+1) | ++ |
| Example 73 | 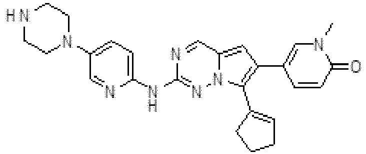 | Following Synthesis Scheme IV | MS-ESI: 469(M+1) | +++ |
| Example 74 | 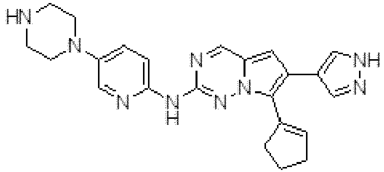 | Following Synthesis Scheme IV | MS-ESI: 428(M+1) | +++ |
| Example 75 | 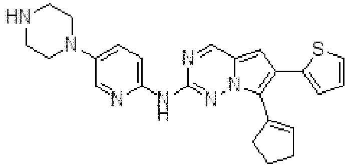 | Following Synthesis Scheme IV | MS-ESI: 444(M+1) | +++ |
| Example 76 | 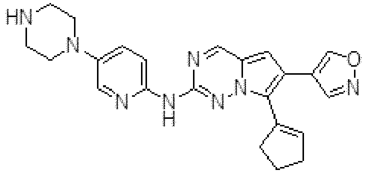 | Following Synthesis Scheme IV | MS-ESI: 429(M+1) | +++ |
| Example 77 | 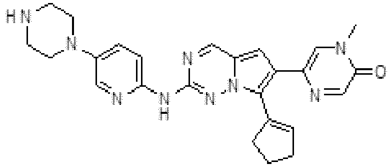 | Following Synthesis Scheme V | MS-ESI: 470(M+1) | ++ |
| Example 78 | 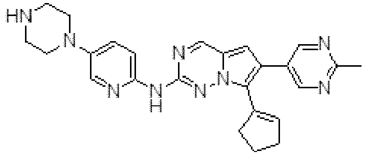 | Following Synthesis Scheme V | MS-ESI: 454(M+1) | +++ |
| Example 79 | 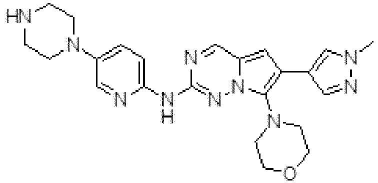 | Following Synthesis Scheme III | MS-ESI: 461(M+1) | +++ |
| Example 80 | 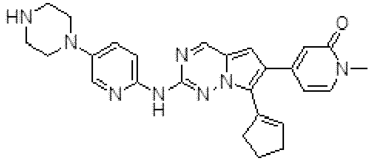 | Following Synthesis Scheme V | MS-ESI: 469(M+1) | +++ |
| Example 81 | 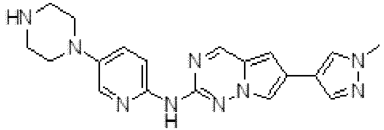 | Following Synthesis Scheme III | MS-ESI: 376(M+1) | ++ |
| Example 82 | 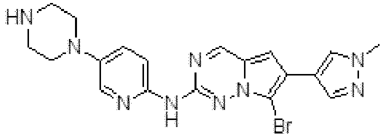 | Following Synthesis Scheme III | MS-ESI: 454(M+1) | ++ |
| Example 83 | 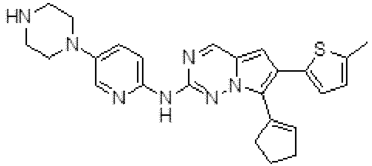 | Following Synthesis Scheme IV | MS-ESI: 458(M+1) | ++ |
| Example 84 | 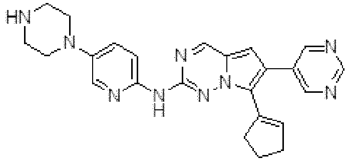 | Following Synthesis Scheme IV | MS-ESI: 440(M+1) | +++ |
| Example 85 | 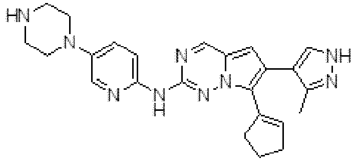 | Following Synthesis Scheme IV | MS-ESI: 442(M+1) | +++ |
| Example 86 | 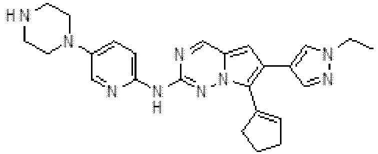 | Following Synthesis Scheme IV | MS-ESI: 456(M+1) | +++ |
| Example 87 | 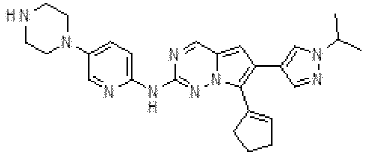 | Following Synthesis Scheme IV | MS-ESI: 470(M+1) | ++ |
| Example 88 | 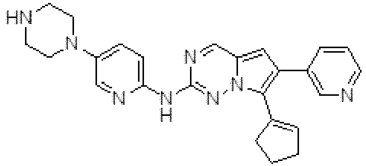 | Following Synthesis Scheme IV | MS-ESI: 439(M+1) | +++ |
| Example 89 | 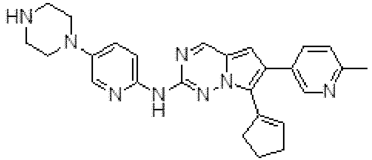 | Following Synthesis Scheme IV | MS-ESI: 453(M+1) | +++ |
| Example 90 | 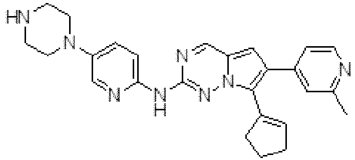 | Following Synthesis Scheme IV | MS-ESI: 453(M+1) | +++ |
| Example 91 | 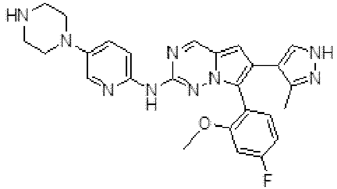 | Following Synthesis Scheme III | MS-ESI: 500(M+1) | ++ |
| Example 92 | 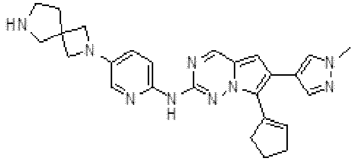 | Following Synthesis Scheme IV | MS-ESI: 468(M+1) | ++ |
| Example 93 | 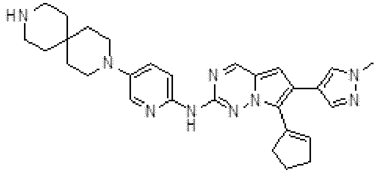 | Following Synthesis Scheme IV | MS-ESI: 510(M+1) | +++ |
| Example 94 | 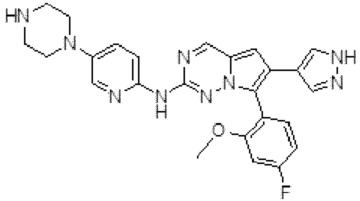 | Following Synthesis Scheme III | MS-ESI: 486(M+1) | +++ |
| Example 95 | 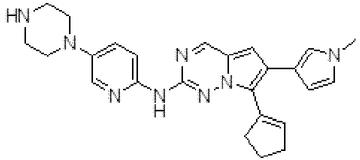 | Following Synthesis Scheme IV | MS-ESI: 441(M+1) | ++ |
| Example 96 | 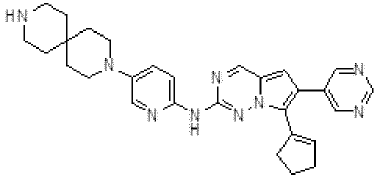 | Following Synthesis Scheme IV | MS-ESI: 508(M+1) | +++ |
| Example 97 | 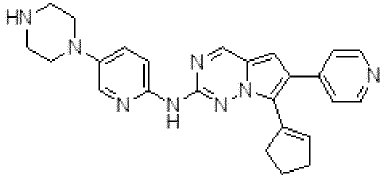 | Following Synthesis Scheme IV | MS-ESI: 439(M+1) | +++ |
| Example 98 | 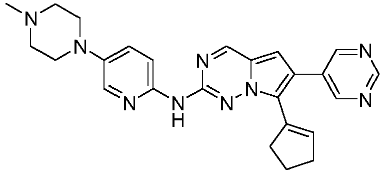 | Following Synthesis Scheme IV | MS-ESI: 454(M+1) | +++ |
| Example 99 | 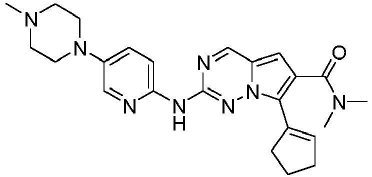 | Following Synthesis Scheme VI | MS-ESI: 447(M+1) | +++ |
| Example 100 | 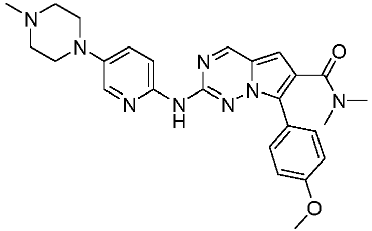 | Following Synthesis Scheme VI | MS-ESI: 487(M+1) | +++ |
| Example 101 | 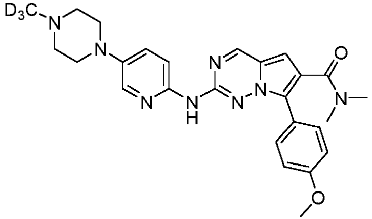 | Following Synthesis Scheme VI | MS-ESI: 490(M+1) | +++ |
| Example 102 | 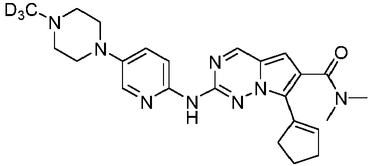 | Following Synthesis Scheme VI | MS-ESI: 450(M+1) | +++ |
| Example 103 | 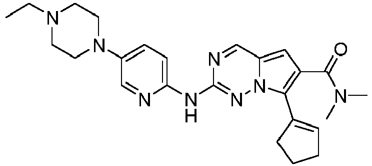 | Following Synthesis Scheme VI | MS-ESI: 461(M+1) | +++ |
| Example 104 | 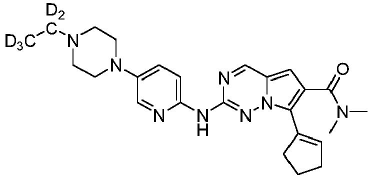 | Following Synthesis Scheme VI | MS-ESI: 466(M+1) | +++ |
| Example 105 | 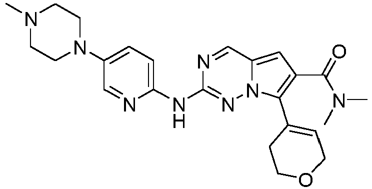 | Following Synthesis Scheme VI | MS-ESI: 463(M+1) | +++ |
| Example 106 | 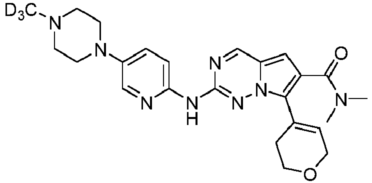 | Following Synthesis Scheme VI | MS-ESI: 466(M+1) | +++ |
| Example 107 | 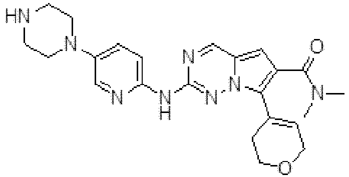 | Following Synthesis Scheme VI | MS-ESI: 449(M+1) | +++ |
| Example 108 | 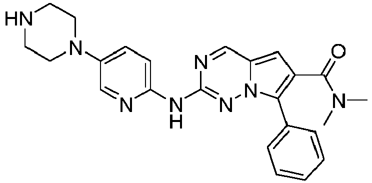 | Following Synthesis Scheme VI | MS-ESI: 443(M+1) | +++ |
| Example 109 | 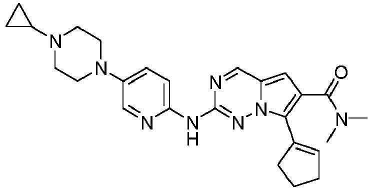 | Following Synthesis Scheme VI | MS-ESI: 473(M+1) | +++ |
| Example 110 | 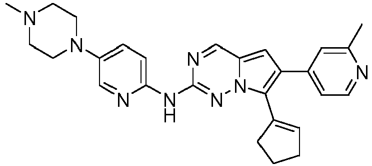 | Following Synthesis Scheme IV | MS-ESI: 467(M+1) | +++ |
| Example 111 | 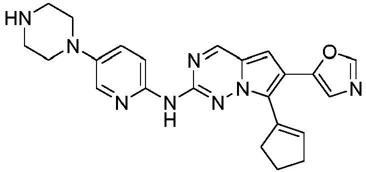 | Following Synthesis Scheme IV | MS-ESI: 429(M+1) | +++ |
| Example 112 | 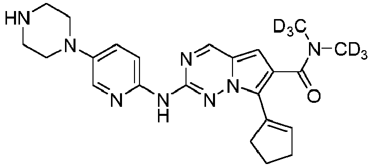 | Following Synthesis Scheme VI | MS-ESI: 439(M+1) | +++ |
| Example 113 | 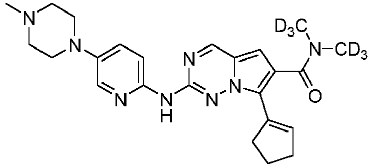 | Following Synthesis Scheme VI | MS-ESI: 453(M+1) | +++ |
| Example 114 | 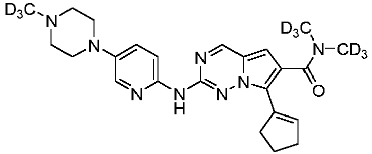 | Following Synthesis Scheme VI | MS-ESI: 456(M+1) | +++ |
| Example 115 | 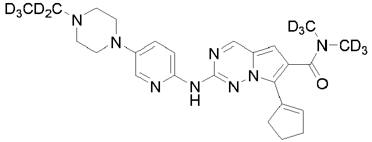 | Following Synthesis Scheme VI | MS-ESI: 472(M+1) | +++ |
| Example 116 | 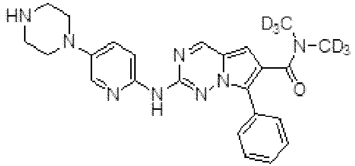 | Following Synthesis Scheme VI | MS-ESI: 449(M+1) | +++ |
| Example 117 | 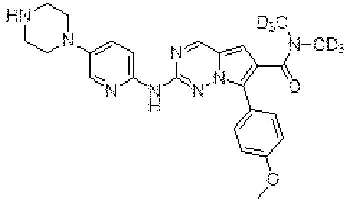 | Following Synthesis Scheme VI | MS-ESI: 479(M+1) | +++ |
| Example 118 | 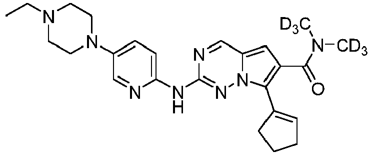 | Following Synthesis Scheme VI | MS-ESI: 467(M+1) | +++ |
| Example 119 | 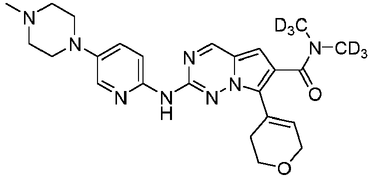 | Following Synthesis Scheme VI | MS-ESI: 469(M+1) | +++ |
| Example 120 | 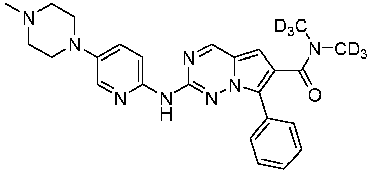 | Following Synthesis Scheme VI | MS-ESI: 463(M+1) | +++ |
| Example 121 | 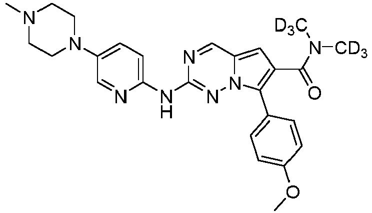 | Following Synthesis Scheme VI | MS-ESI: 493(M+1) | +++ |
| Example 122 | 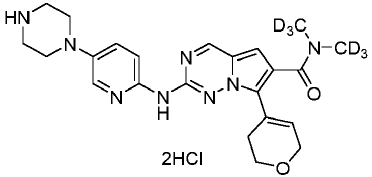 | Following Synthesis Scheme VI | MS-ESI: 455(M+1) | +++ |
| Example 123 | 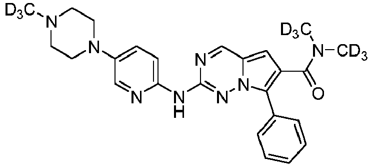 | Following Synthesis Scheme VI | MS-ESI: 466(M+1) | +++ |
| Example 124 | 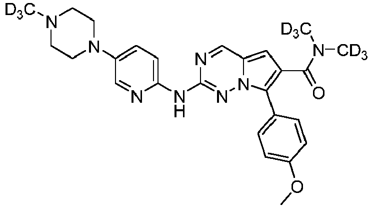 | Following Synthesis Scheme VI | MS-ESI: 496(M+1) | +++ |
| Example 125 | 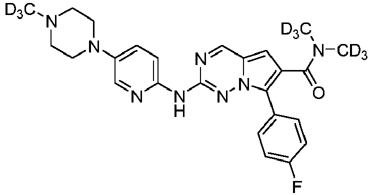 | Following Synthesis Scheme VI | MS-ESI: 484(M+1) | +++ |
| Example 126 | 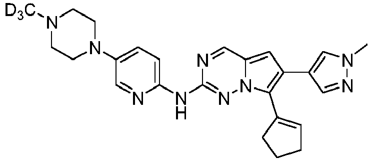 | Following Synthesis Scheme IV | MS-ESI: 459(M+1) | +++ |
| Example 127 | 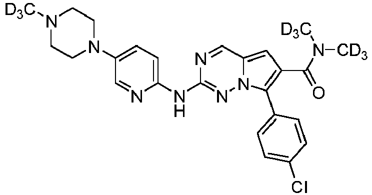 | Following Synthesis Scheme VI | MS-ESI: 500(M+1) | +++ |
| Reference Example 128 | 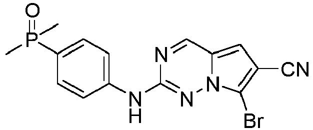 | Following Synthesis Scheme II | MS-ESI: 390(M+1) | + |
| Reference Example 129 | 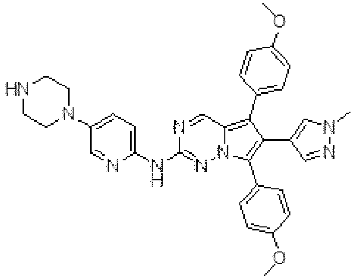 | Following Synthesis Scheme I or III | MS-ESI: 588(M+1) | - |
| Reference Example 130 | 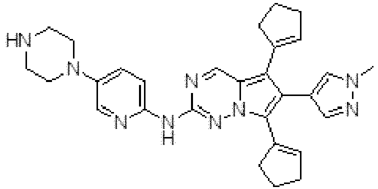 | Following Synthesis Scheme I or III | MS-ESI: 508(M+1) | + |
| Reference Example 131 | 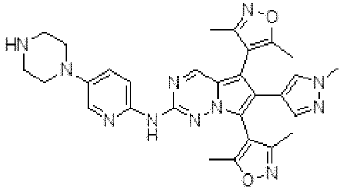 | Following Synthesis Scheme III | MS-ESI: 566(M+1) | - |
| Reference Example 132 | 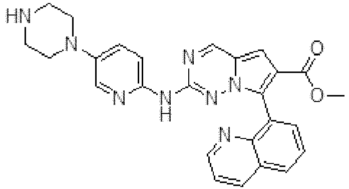 | Following Synthesis Scheme VI | MS-ESI: 481(M+1) | - |
| Reference Example 133 | 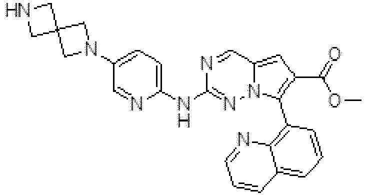 | Following Synthesis Scheme VI | MS-ESI: 493(M+1) | - |
| Reference Example 134 | 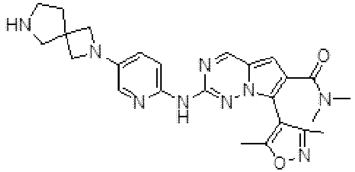 | Following Synthesis Scheme VI | MS-ESI: 488(M+1) | + |

As can be seen from Table 1, the Example compounds within the protection scope of the present invention can cause good inhibition on the activity of CDK6 kinase.

### Test Example 2: Cell Viability assay

An *in vitro* cell viability assay was conducted using some Example compounds following the steps below:
1. After recovery, MCF-7 cells (obtained from the Shanghai Cell Bank of the Chinese Academy of Sciences) were cultured at 37 °C, 5% CO₂, and 95% humidity.
2. 8 compound solutions at different concentrations were prepared for each Example compound. 50 µL of the compound solution at each concentration was added to a 96-well black plate.
3. The cell concentration was adjusted to about 30,000 cells/mL. 100 µL of the cell suspension was added to the 96-well plate, and mixed well to a final cell density of about 3,000 cells/well.
4. The 96-well plate was incubated at 37 °C, 5% CO₂, and 95% humidity for 168 h, i.e., 7 days.
5. The cell survival rate was measured by the method of CellTiter-Glo^{®} Luminescent Cell Viability Assay (Promega, Cat#G7572). The fluorescence data were read on a microplate reader (EnVision^{™}, a multi-function microplate detector, PerkinElmer).
6. The obtained fluorescence data were analyzed using the GraphPad Prism software. The cell survival rate was calculated for each Example compound at each concentration, and the IC₅₀ interval of each Example compound was determined.

The IC₅₀ values of the Example compounds are listed in Table 2 below, where "+++" represents IC₅₀ < 500 nM; "++" represents 500 nM ≤ IC₅₀ < 2500 nM; "+" represents 2500 nM <IC₅₀<10000 nM; and "-" represents more than 10000 nM.

**Table 2. Inhibition of the Example compounds on MCF-7 cells in vitro**

| No. | IC₅₀ (MCF-7) |
|---|---|
| Example 1 | +++ |
| Example 2 | +++ |
| Example 3 | +++ |
| Example 4 | +++ |
| Example 5 | +++ |
| Example 6 | +++ |
| Example 7 | +++ |
| Example 8 | ++ |
| Example 9 | +++ |
| Example 10 | +++ |
| Example 11 | +++ |
| Example 12 | +++ |
| Example 13 | +++ |
| Example 14 | +++ |
| Example 15 | +++ |
| Example 16 | ++ |
| Example 17 | +++ |
| Example 18 | +++ |
| Example 19 | ++ |
| Example 20 | ++ |
| Example 21 | ++ |
| Example 22 | +++ |
| Example 23 | +++ |
| Example 24 | +++ |
| Example 25 | +++ |
| Example 26 | +++ |
| Example 27 | + |
| Example 28 | +++ |
| Example 29 | +++ |
| Example 30 | + |
| Example 31 | +++ |
| Example 32 | +++ |
| Example 33 | +++ |
| Example 34 | +++ |
| Example 35 | ++ |
| Example 37 | ++ |
| Example 38 | +++ |
| Example 39 | +++ |
| Example 40 | +++ |
| Example 41 | +++ |
| Example 42 | +++ |
| Example 43 | ++ |
| Example 44 | +++ |
| Example 45 | ++ |
| Example 46 | +++ |
| Example 47 | + |
| Example 49 | ++ |
| Example 50 | + |
| Example 51 | ++ |
| Example 52 | + |
| Example 53 | ++ |
| Example 54 | ++ |
| Example 55 | +++ |
| Example 57 | ++ |
| Example 58 | + |
| Example 59 | ++ |
| Example 60 | + |
| Example 65 | + |
| Example 68 | + |
| Example 69 | + |
| Example 71 | ++ |
| Example 72 | ++ |
| Example 73 | ++ |
| Example 74 | +++ |
| Example 75 | ++ |
| Example 76 | + |
| Example 78 | + |
| Example 81 | + |
| Example 82 | +++ |
| Example 83 | ++ |
| Example 84 | +++ |
| Example 85 | +++ |
| Example 86 | +++ |
| Example 87 | +++ |
| Example 88 | +++ |
| Example 89 | +++ |
| Example 90 | +++ |
| Example 91 | + |
| Example 92 | ++ |
| Example 93 | +++ |
| Example 94 | +++ |
| Example 95 | + |
| Example 96 | ++ |
| Example 97 | +++ |
| Example 98 | ++ |
| Example 99 | +++ |
| Example 100 | +++ |
| Example 101 | +++ |
| Example 102 | +++ |
| Example 103 | +++ |
| Example 104 | +++ |
| Example 105 | +++ |
| Example 106 | +++ |
| Example 107 | +++ |
| Example 108 | +++ |
| Example 109 | +++ |
| Example 110 | +++ |
| Example 111 | +++ |
| Example 112 | +++ |
| Example 113 | +++ |
| Example 114 | +++ |
| Example 115 | +++ |
| Example 116 | +++ |
| Example 117 | +++ |
| Example 118 | +++ |
| Example 119 | +++ |
| Example 120 | +++ |
| Example 121 | +++ |
| Example 122 | +++ |
| Example 123 | +++ |
| Example 124 | +++ |
| Example 125 | +++ |
| Example 126 | +++ |
| Example 127 | +++ |
| Reference Example 128 | - |
| Reference Example 131 | - |
| Reference Example 132 | - |
| Reference Example 133 | - |
| Reference Example 134 | - |

As can be seen from the above table, the compounds of the present invention can achieve an excellent inhibitory effect on MCF-7 cells.

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by reference in their entirety for all purposes.

## Claims

1. A compound of Formula (I): 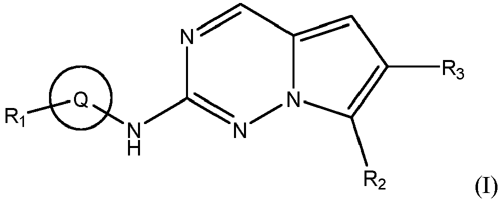 wherein,
Q is an optionally substituted 6- to 18-membered arylene group or an optionally substituted 5- to 18-membered heteroarylene group, wherein, when substituted, the substituent is selected from the group consisting of halo, hydroxy, C₁₋₆alkyl, C₁₋₆alkoxy, and halo-C₁₋₆alkyl;
R₁ is an optionally substituted 3- to 8-membered heterocyclyl group, an optionally substituted 6- to 14-membered fused heterocyclyl group, or an optionally substituted 6- to 12-membered spiro heterocyclyl group, wherein, when substituted, the substituent is selected from the group consisting of halo, hydroxy, C₁₋₆alkyl, C₁₋₆alkoxy, and halo-C₁₋₆alkyl;
R₂ is H, halo, an optionally substituted 3- to 10-membered cycloalkenyl group, an optionally substituted 3- to 10-membered heterocycloalkenyl group, an optionally substituted 3- to 10-membered cycloalkyl group, an optionally substituted 3- to 10-membered heterocycloalkyl group, an optionally substituted 6- to 18-membered aryl group, or an optionally substituted 5- to 18-membered heteroaryl group, wherein, when substituted, the substituent is selected from the group consisting of halo, hydroxy, C₁₋₆alkyl, C₁₋₆alkoxy, halo-C₁₋₆alkyl, C₁₋₆alkoxy-C₁₋₆alkoxy, and oxo;
R₃ is H, CN, -C(=O)-NR₄R₅, an optionally substituted 6- to 18-membered aryl group, an optionally substituted 5- to 18-membered heteroaryl group, or an optionally substituted 5- to 8-membered lactam group, wherein, when substituted, the substituent is selected from the group consisting of halo, hydroxy, C₁₋₆alkyl, C₁₋₆alkoxy, and halo-C₁₋₆alkyl;
R₄ and R₅ are each independently methyl or ethyl; and
when R₃ is -C(=O)-NR₄R₅, R₂ is an optionally substituted 3- to 10-membered cycloalkenyl group, an optionally substituted 3- to 10-membered heterocycloalkenyl group, an optionally substituted 3-to 10-membered heterocycloalkyl group connected to the non-R₂ structure in Formula (I) at a N atom, or an optionally substituted 6- to 18-membered aryl group, wherein, when substituted, the substituent is selected from the group consisting of halo, hydroxy, C₁₋₆alkyl, C₁₋₆alkoxy, halo-C₁₋₆alkyl, C₁₋₆alkoxy-C₁₋₆alkoxy, and oxo,
or a pharmaceutically acceptable salt thereof.

2. The compound of claim 1, or a pharmaceutically acceptable salt thereof, wherein,
Q is optionally substituted phenylene or optionally substituted pyridinylene, wherein, when substituted, the substituent is selected from the group consisting of halo, hydroxy, C₁₋₆alkyl, C₁₋₆alkoxy, and halo-C₁₋₆alkyl;
R₁ is an optionally substituted 3- to 8-membered heterocyclyl group, an optionally substituted 6- to 14-membered fused heterocyclyl group, or an optionally substituted 6- to 12-membered spiro heterocyclyl group, wherein, when substituted, the substituent is selected from C₁₋₆alkyl;
R₂ is selected from the group consisting of a halogen atom, optionally substituted cyclopentenyl, optionally substituted cyclohexenyl, optionally substituted cyclopentyl, optionally substituted cyclohexyl, optionally substituted oxacyclohexenyl, optionally substituted azacyclohexenyl, optionally substituted oxolanyl, optionally substituted azacyclopentyl, optionally substituted oxacyclohexyl, optionally substituted azacyclohexyl, optionally substituted phenyl, optionally substituted naphthyl, optionally substituted pyridinyl, optionally substituted thienyl, optionally substituted pyrazolyl, optionally substituted oxazolyl, optionally substituted isoxazolyl, and optionally substituted quinolyl, wherein, when substituted, the substituent is selected from the group consisting of halo, hydroxy, C₁₋₆alkyl, C₁₋₆alkoxy, halo-C₁₋₆alkyl, and oxo;
R₃ is H, -CN, -C(=O)-NR₄R₅, optionally substituted phenyl, naphthyl, pyrazolyl, pyridinyl, thienyl, oxazolyl, isoxazolyl, pyrimidinyl, imidazolyl, pyrrolyl, 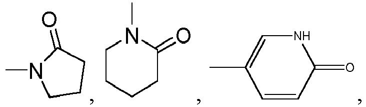 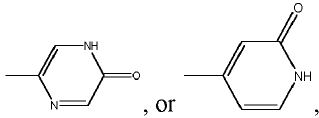 wherein, when substituted, the substituent is selected from the group consisting of halo, hydroxy, C₁₋₆alkyl, C₁₋₆alkoxy, and halo-C₁₋₆alkyl; and
both R₄ and R₅ are methyl.

3. The compound of claim 1 or 2, or a pharmaceutically acceptable salt thereof, wherein Q is phenylene or

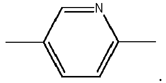

4. The compound of any one of claims 1 to 3, or a pharmaceutically acceptable salt thereof, wherein R₁ is an optionally substituted group as below, wherein, when substituted, the substituent is selected from C₁₋₆alkyl:

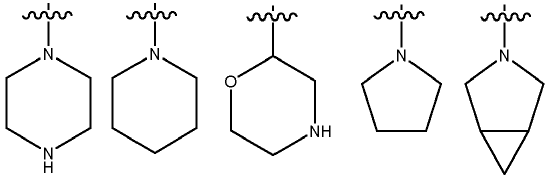

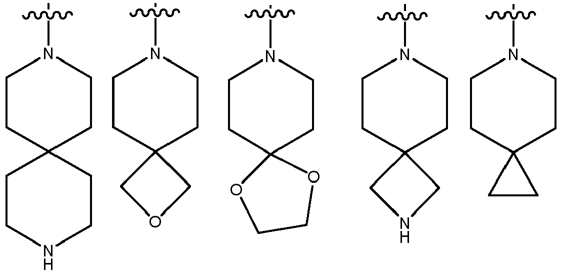

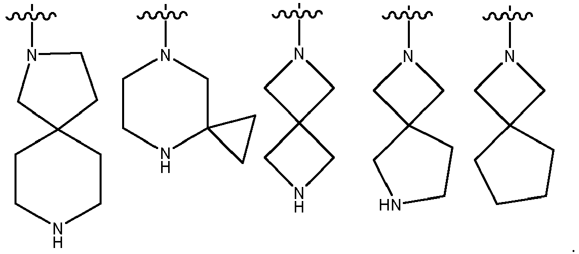

5. The compound of claim 4, or a pharmaceutically acceptable salt thereof, wherein the C₁₋₆alkyl is methyl or ethyl.

6. The compound of any one of claims 1 to 5, or a pharmaceutically acceptable salt thereof, wherein R₂ is halo or an optionally substituted group as below, wherein, when substituted, the substituent is selected from C₁₋₆alkyl:

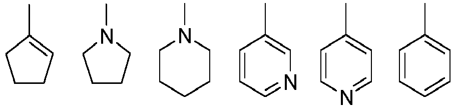

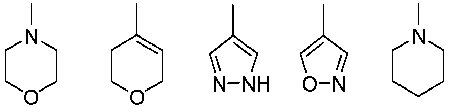

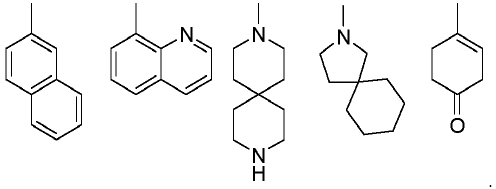

7. The compound of claim 1, or a pharmaceutically acceptable salt thereof, wherein the compound is selected from the group consisting of: 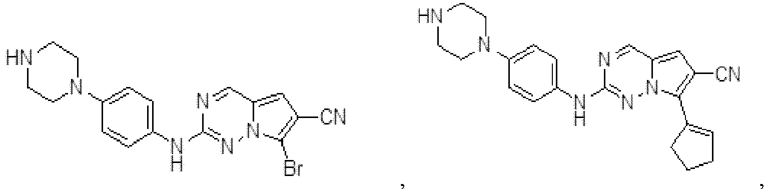 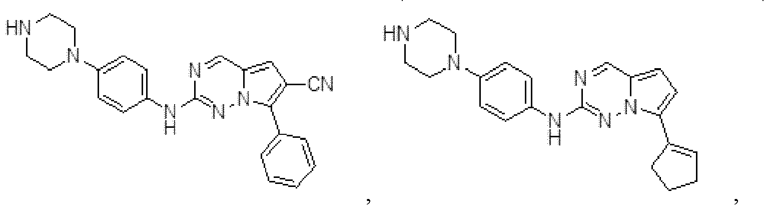 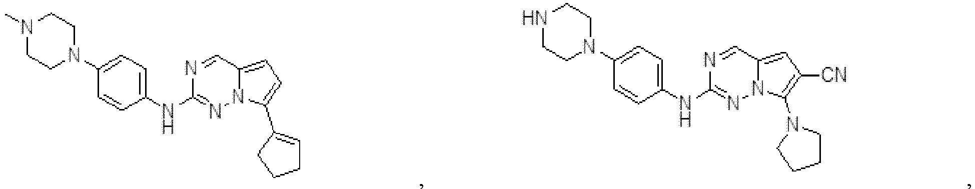 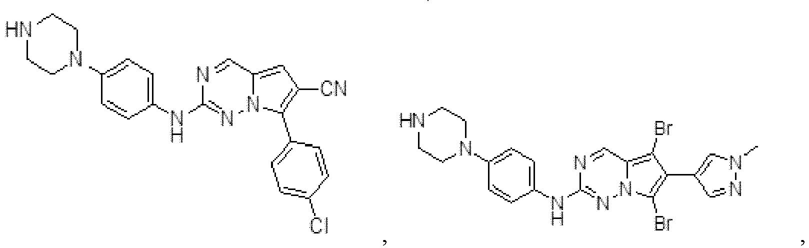 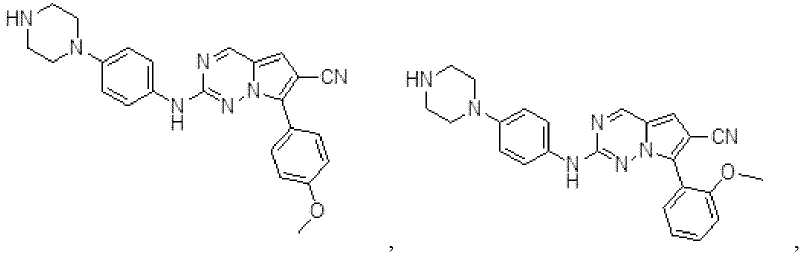 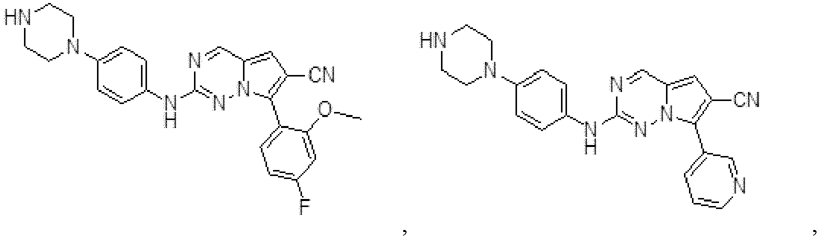 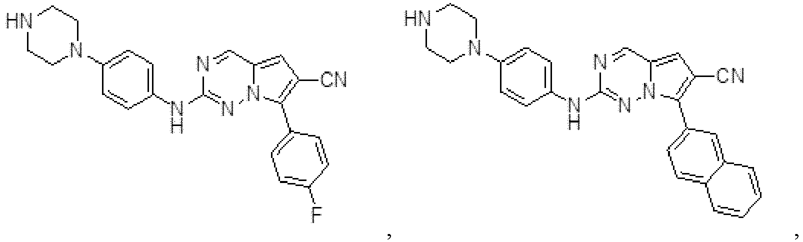 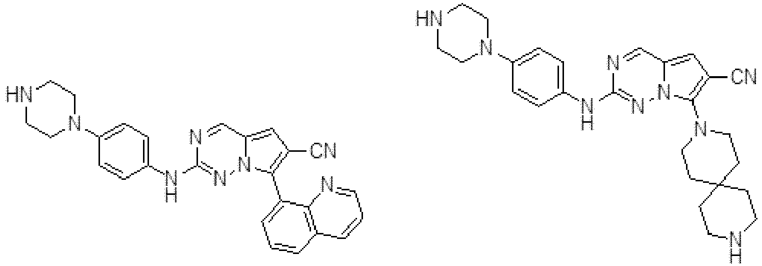 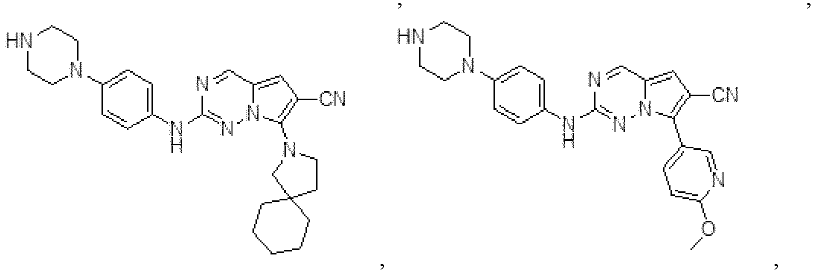 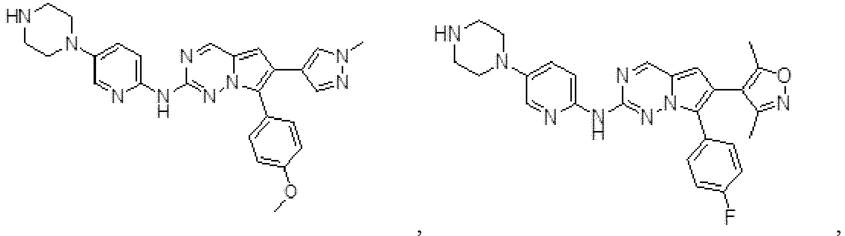 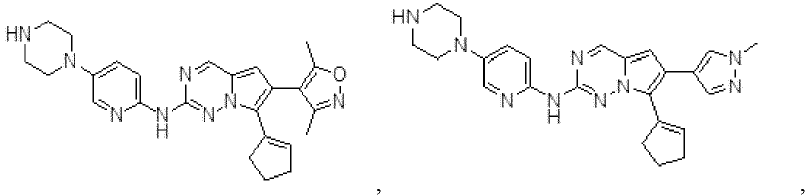 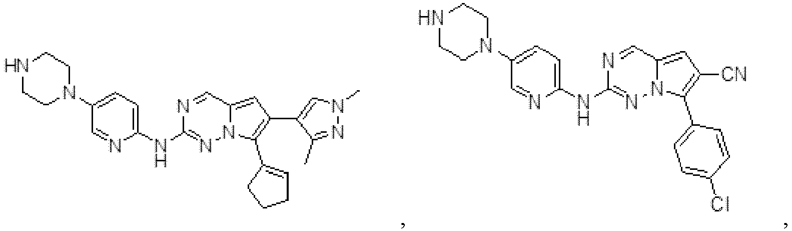 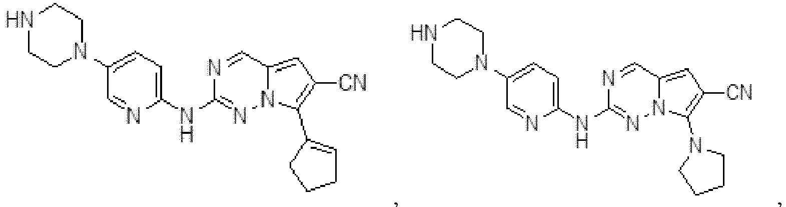 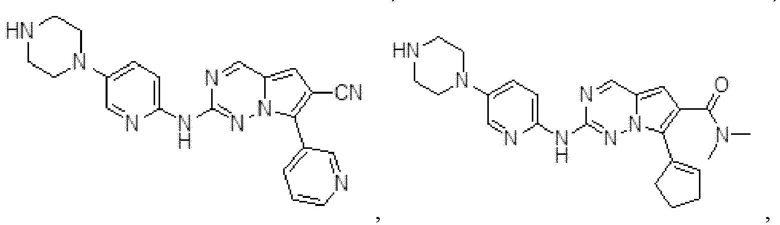 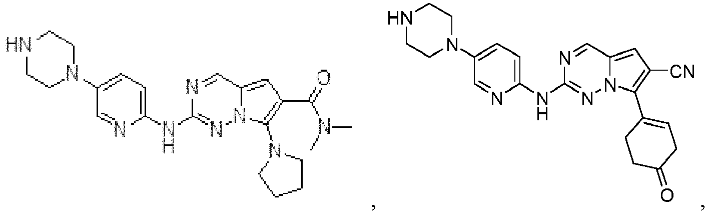 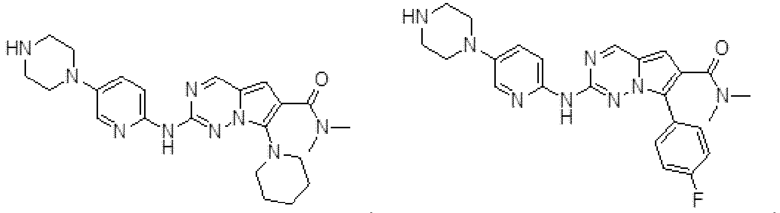 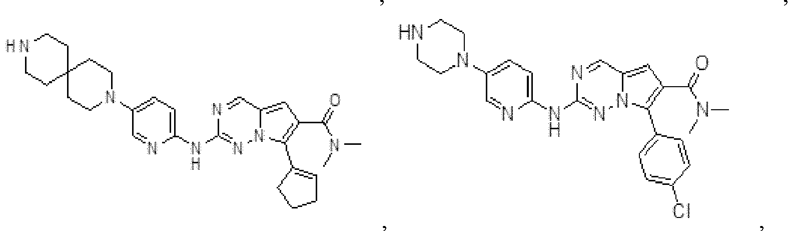 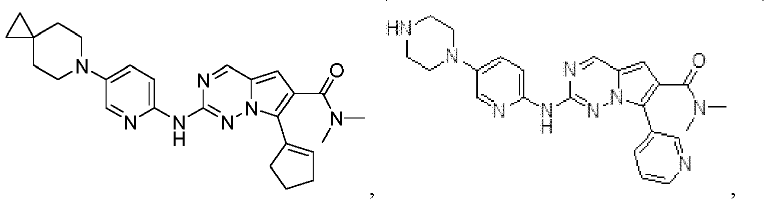 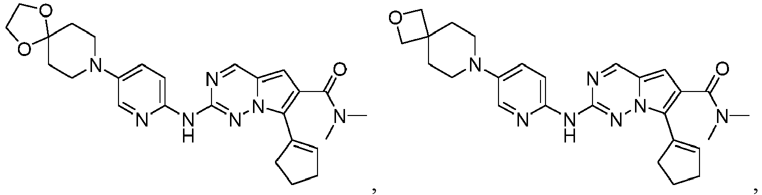 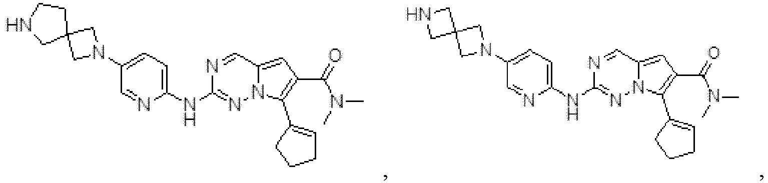 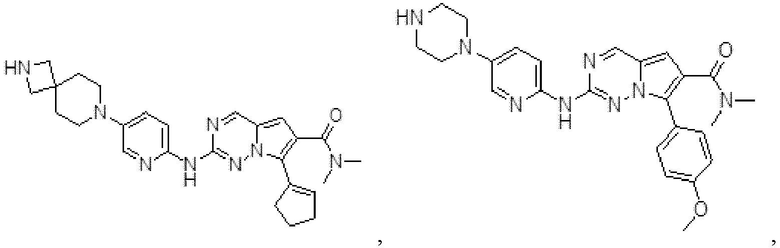 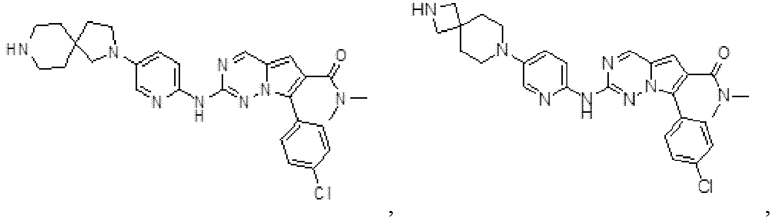 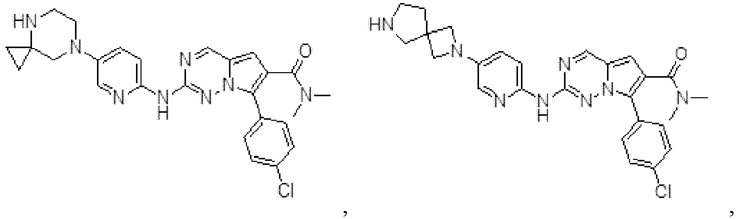 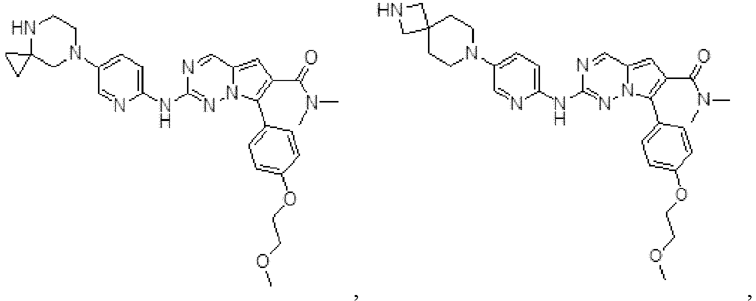 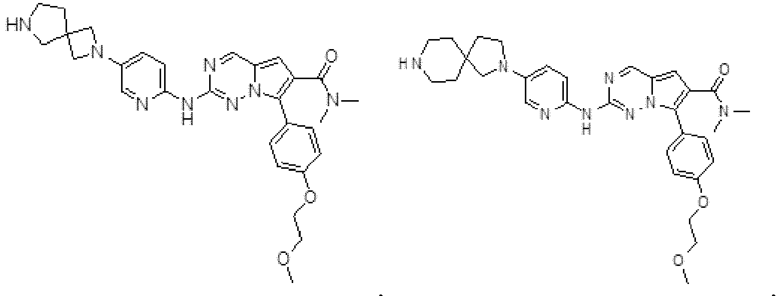 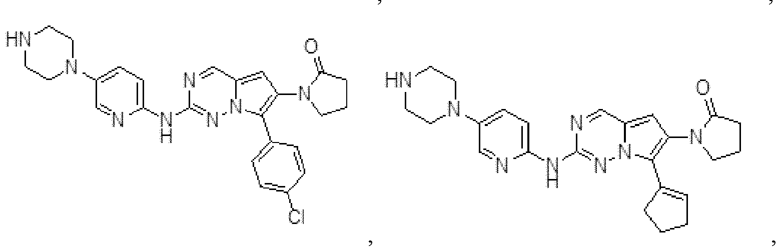 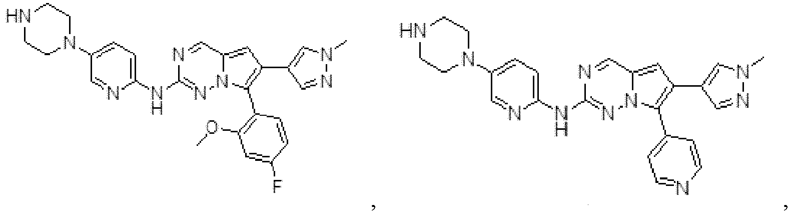 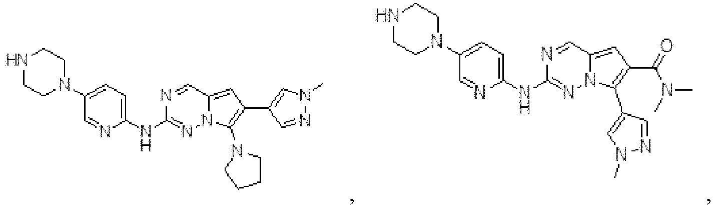 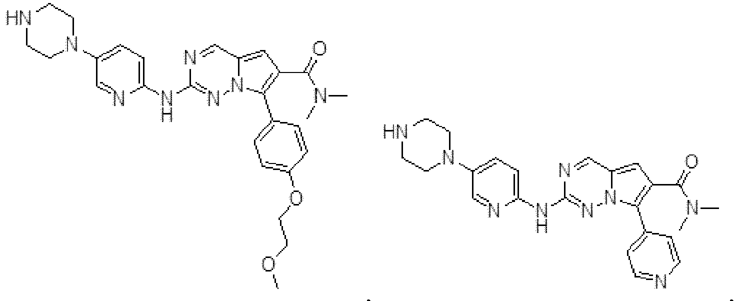 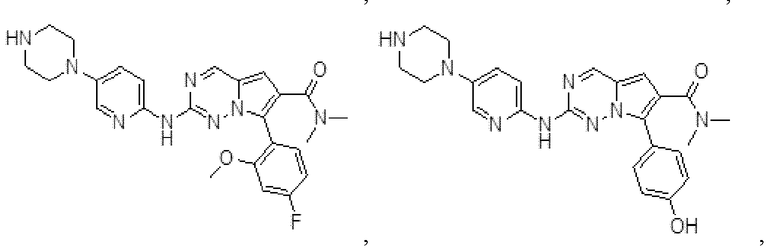 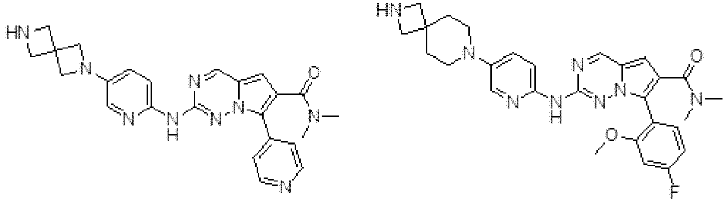 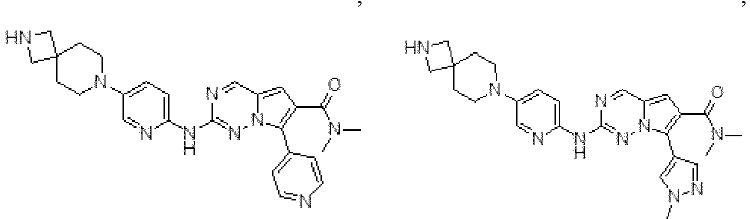 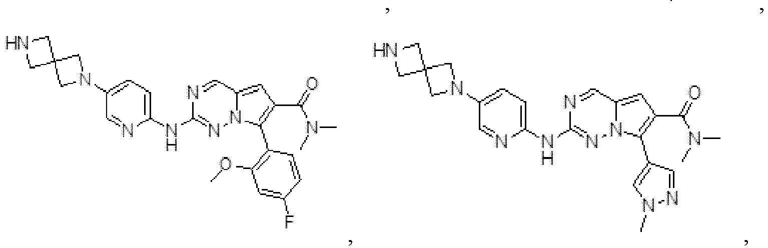 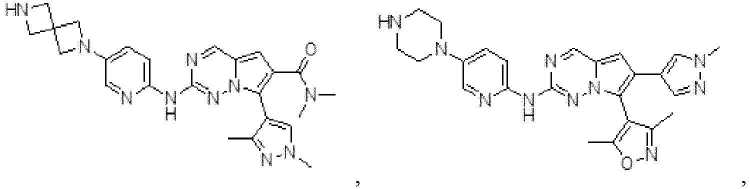 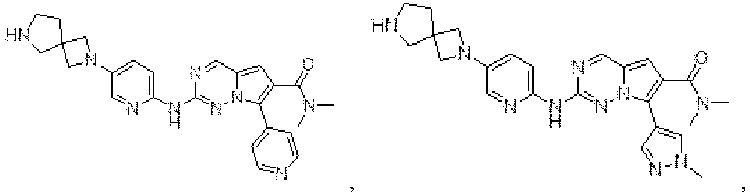 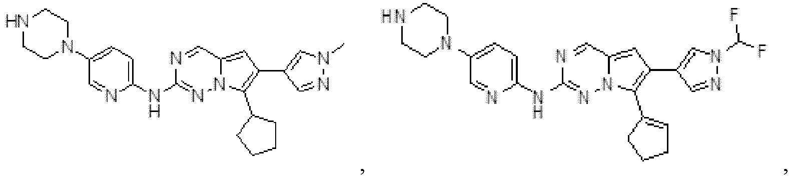 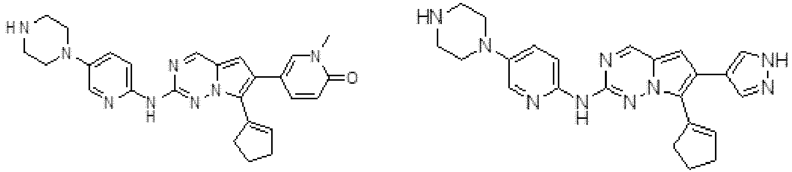 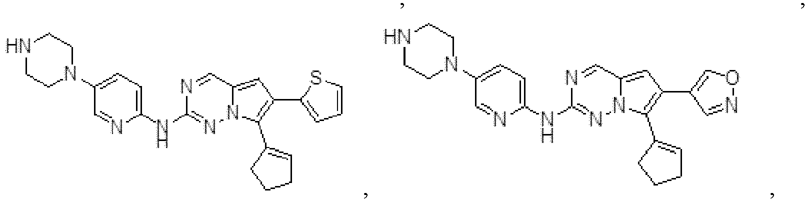 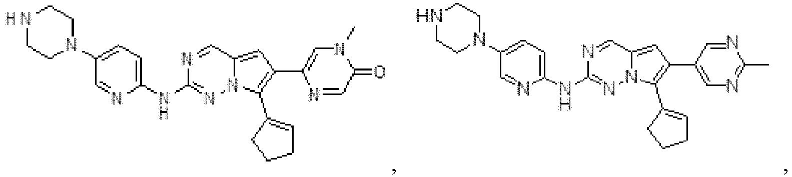 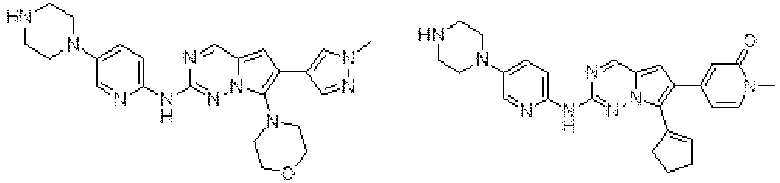 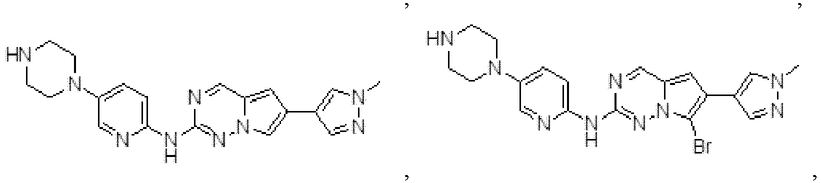 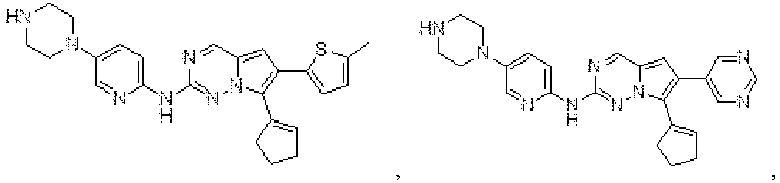 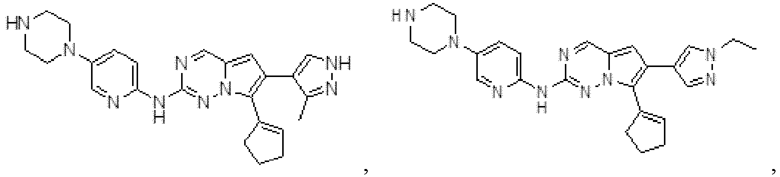 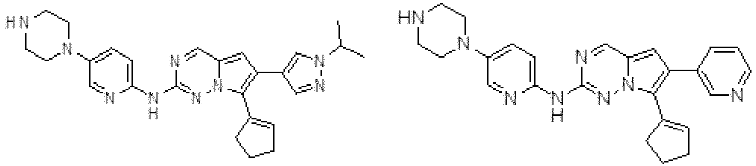 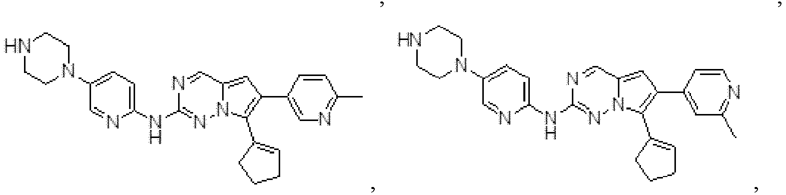 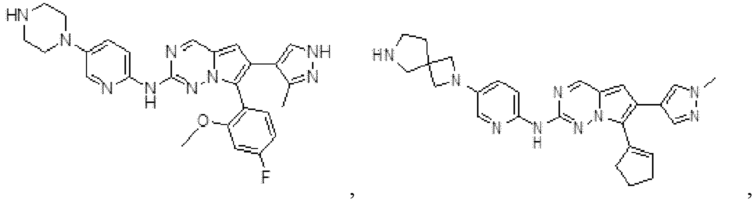 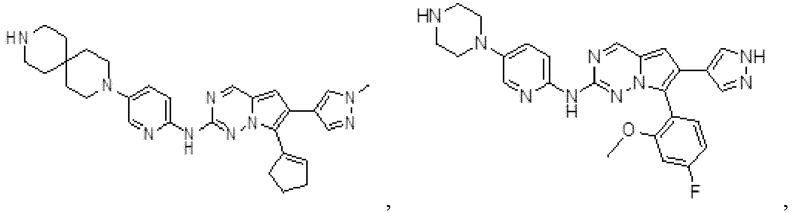 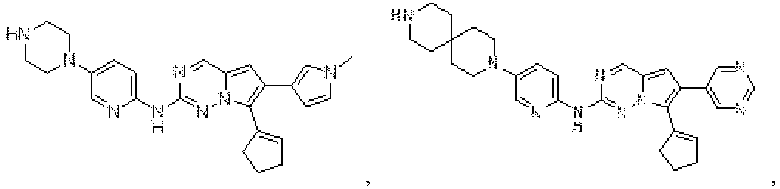 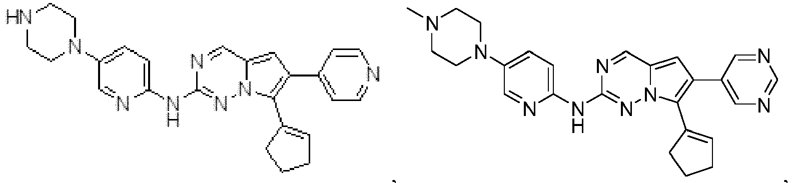 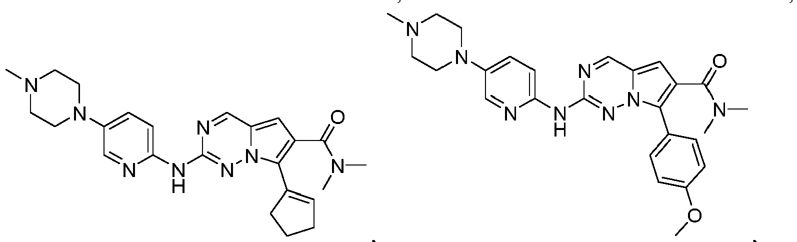 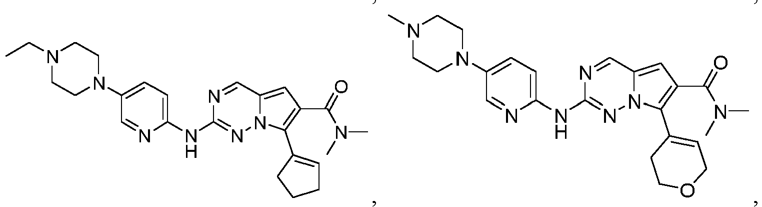 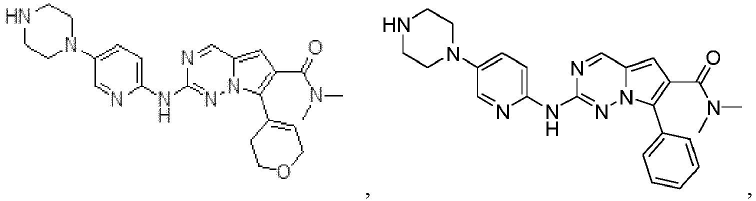 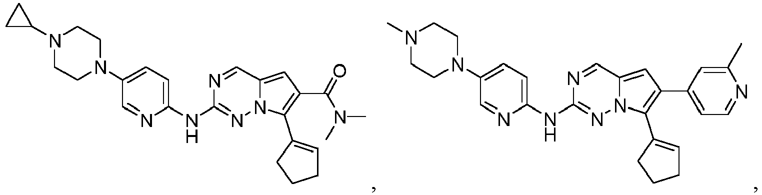 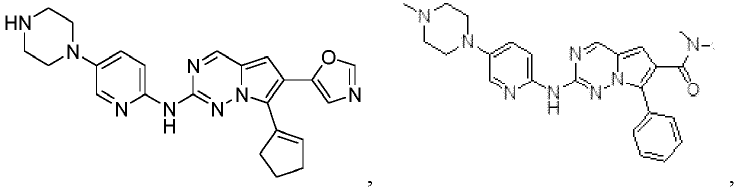 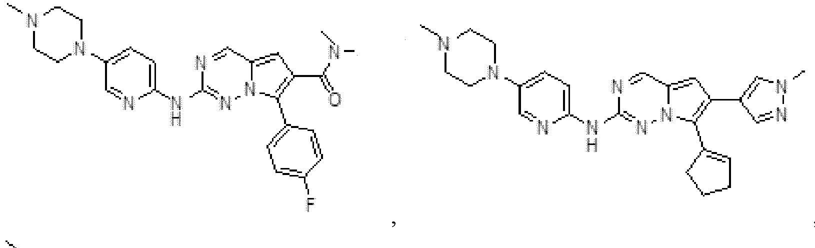 ,and

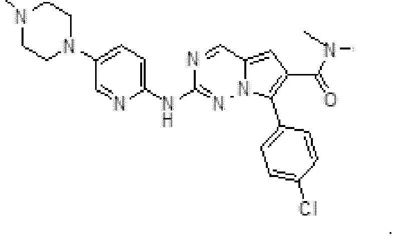

8. The compound of any one of claims 1 to 7 for inhibiting the activity of CDK4/6 kinase.

9. A pharmaceutical composition comprising a therapeutically effective amount of a compound of any one of claims 1 to 8, and a pharmaceutically acceptable carrier or excipient.

10. Use of the compound of any one of claims 1 to 8, or a pharmaceutically acceptable salt thereof, in the manufacture of a medicament for treating and/or preventing a cancer-related disease mediated by CDK4/6 kinase, wherein the cancer-related disease is selected from brain tumor, lung cancer, squamous cell carcinoma, bladder cancer, stomach cancer, ovarian cancer, peritoneal cancer, pancreatic cancer, breast cancer, head and neck cancer, cervical cancer, endometrial cancer, rectal cancer, liver cancer, kidney cancer, esophageal adenocarcinoma, esophageal squamous cell carcinoma, prostate cancer, female reproductive tract cancer, carcinoma *in situ*, lymphoma, neurofibroma, thyroid cancer, bone cancer, skin cancer, brain cancer, colon cancer, testicular cancer, gastrointestinal stromal tumor, prostate tumor, mast cell tumor, multiple myeloma, melanoma, glioma, or sarcoma.
